# EUROPEAN PATENT APPLICATION

(11) **EP 2 290 361 A1**
(43) Date of publication of application: **02.03.2011**
(21) Application number: 10012955.0
(22) Date of filing: 16.02.2007
(51) Int. Cl.: G01N 33/50, G01N 33/574, C07K 16/28

(54) **Methods for selecting a colorectal carcinoma treatment**

(30) Priority: 16.02.2006 US 774563 P
(62) Divisional of application: 07757161.0
(71) Applicant: Ventana Medical Systems, Inc., Tucson, Arizona 85755 (US)
(72) Inventor: Pestano, Gary, Anthony, Oro Valley AZ 85737 (US); Samadzadeh, Linda, Kay, Tacoma WA 98407 (US)
(74) Representative: Müller-Boré & Partner Patentanwälte

(57) **Abstract**

The present invention relates to a method for identifying a colorectal cancer tumor responsive to a chemotherapeutic agent.

## Description

This application claims priority to U.S. provisional application Serial No. 60/774,563 filed February 16, 2006, which is incorporated by reference herein.

### BACKGROUND OF THE INVENTION

### 1. Field of the Invention

This invention relates to reagents and methods for assessing colorectal cancer progression in an individual. More particularly, the invention provides said reagents and methods for determining or diagnosing the progression or pathological staging of a cancer in order to more accurately tailor therapy to an individual. The invention also relates to immunological reagents and methods for monitoring and analyzing biological samples for quantifying expression and activation of any one or informative combination of biomarkers of the EGFR pathway including EGFR, PTEN, pHER1, pAKT, pERK, pMEK and Ki67.

### 2. Background of the Invention

A primary goal of cancer therapy is to selectively kill or inhibit uncontrolled growth of malignant cells while not adversely affecting normal cells. Traditional chemotherapeutic drugs are highly cytotoxic agents that preferably have greater affinity for malignant cells than for normal cells, or at least preferentially affect malignant cells based on their high rate of cell growth and metabolic activity. However, these agents have not turned out to be "magic bullets" and often harm normal cells as well as cancer cells; paradoxically, certain cancers develop resistance to said chemotherapeutic agents that is not developed by normal cells. Cancer treatment therapies that can target the malignant cells and spare the normal cells, referred to as targeted therapies, are a new wave of cancer chemotherapeutics. Such new approaches are particularly relevant to treating solid tumor cancers, which remain chronic conditions needing flexible and responsive treatments with less side-effects, and their development needs to be investigated.

Generally, targeted cancer therapies attempt to block growth and spread of cancer cells by interfering with molecules or intracellular pathways that are specific to carcinogenesis and thus spare non-cancer cells. These agents work in contradistinction to traditional chemotherapeutic or chemopreventive agents that are used to produce growth arrest, terminal differentiation and cell death of the cancerous or precancerous cells but can interrupt the development of normal cells as well. However, robust diagnostic candidate biomarkers for targeted therapies have been difficult to develop, due in part to a diversity of ligands and receptors expressed by both normal and cancer cells, and resulting variable outcomes from receptor signaling.

Several signaling pathways have emerged as important targets for understanding and treating oncogenesis; these include growth factor signal transduction pathways. The growth factor pathways regulate cell growth and metabolism in response to intracellular and environmental cues. These signaling pathways are often altered or dysregulated in cancer, resulting in a phenotype of uncontrolled growth and invasion of surrounding tissue.

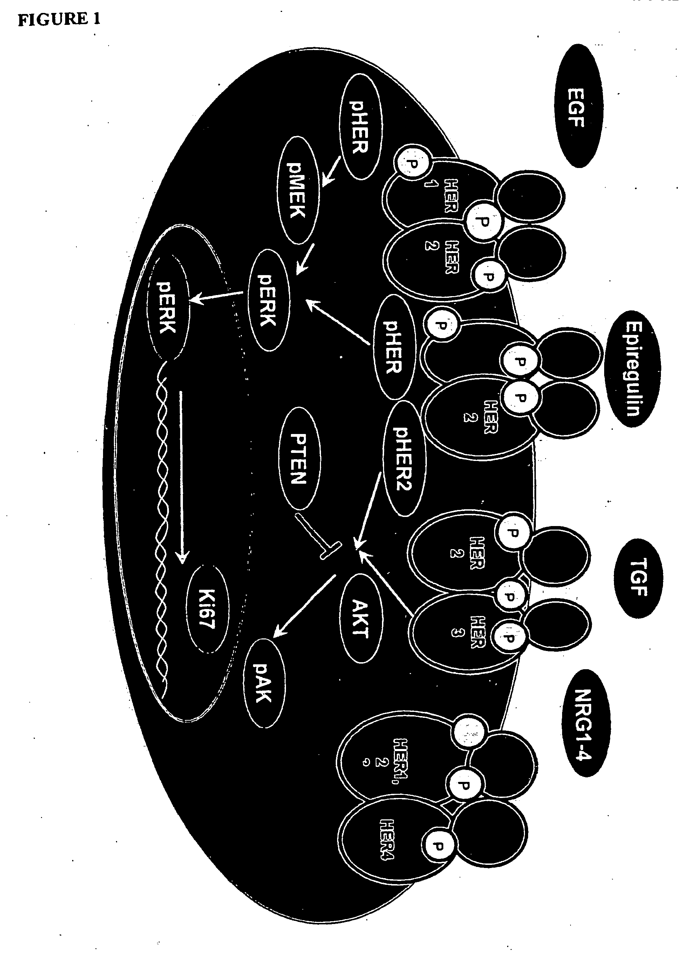

A key determinant of cell growth and a target of active research in cancer diagnosis and treatment is the epidermal growth factor (EGF) and its receptor (EGFR). EGF is a growth factor that activates protein-receptor tyrosine kinase (RTK) activity to initiate a signal transduction cascade resulting in changes in cell growth, proliferation and differentiation. EGF and its downstream targets (illustrated in **FIGURE 1**), including ras/raf, mek, and erk, are known to be involved in the pathogenesis and progression of different cancers. This pathway and its signaling molecules provide attractive targets for therapeutic intervention and such approaches are in development (Stadler, 2005, Cancer, 104:2323-33; Normanno, et al., 2006, Gene, 366:2-16).

Agents that target EGF and its receptor include bevacizumab, PTK787, SU011248 and BAY 43-9006. The BAY 43-9006 has also been shown to inhibit the downstream targets in the EGF pathway including raf, mek and erk (Stadler, 2005 *Cancer, Id.).* This receptor system has also been implicated in the development and progression of a number of human solid tumors including lung, breast, prostate, colon, ovary, head and neck. HER1/EGFR is a member of a family of four receptors {EGFR (also called HER1 or ErbB1), ErbB2 (HER2/neu), ErbB3 (HER3), and ErbB4 (HER4)}. The receptors reside in the cell plasma membrane and consist of an external ligand binding domain, a transmembrane domain, and an internal tyrosine kinase domain. Binding of the ligand triggers receptor dimerization and autophosphorylation of the internal receptor domain. This initiates a cascade of cellular reactions that influences cell division and many other aspects of cell growth. Heightened activity at the EGF receptor, whether caused by increased ligand concentration, receptor numbers, or by receptor mutation can lead to increased cell proliferation. There is now significant evidence to show that the BER1/EGFR system may mediate establishment and progression of a variety of solid tumors.

While studies have examined the role of the EGF pathway in control and treatment of cancer there is little known about changes in this pathway during tumor progression and metastasis. Subtle alterations in the downstream effectors in the EGFR pathway may provide unique and flexible targets for therapeutic intervention based on, *inter alia,* tumor type, stage and individual composition. Such variation could have a significant impact on the diagnosis and treatment of malignancies.

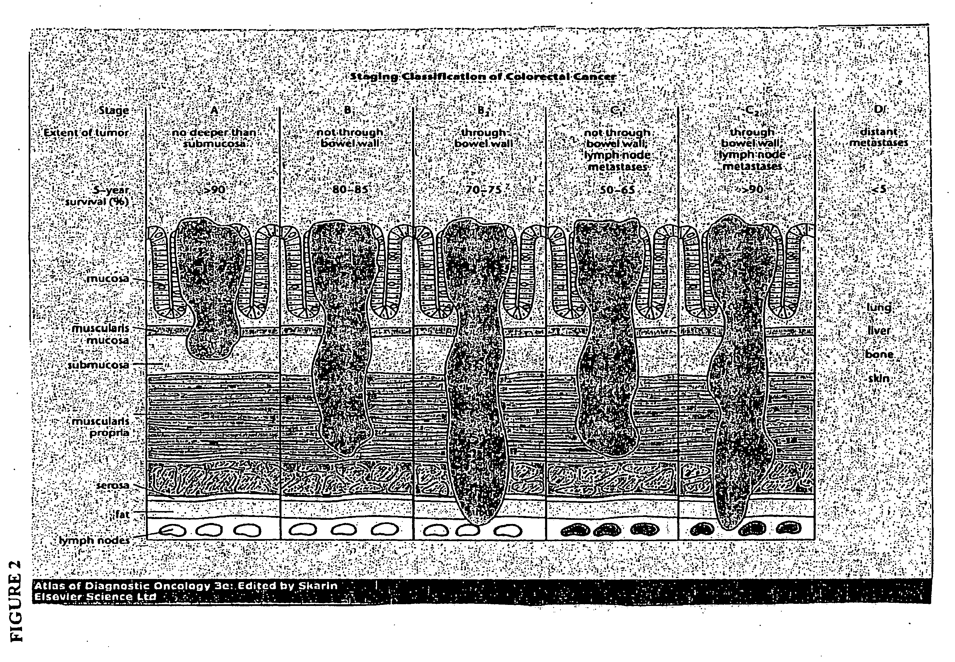

Traditional therapeutic cancer regimens have been developed based upon results from large-scale trials and rely upon predictive outcomes for a wide variety of patients and tumors. The capacity to tailor therapies to the individual patient, tumor, and pathological staging (shown in **FIGURE 2**) may provide more efficacious treatments for malignancies with less side-effects. Furthermore, the ability to monitor the progression of the cancer treatment and adjust the therapy accordingly would allow for a more rapid reaction to individual differences in response to therapeutic regimens that have been previously developed using data from a wide group of patients.

There exists a need in the art to develop diagnostic biomarkers to allow for screening and rapid detection of changes in various intracellular signaling molecules before and during cancer treatment in order to quickly diagnose cancer stage and monitor the effects of treatment directed against the EGFR pathway. There is also a need for improved identification methods of inhibitors directed towards the EGFR pathway.

### SUMMARY OF THE INVENTION

The invention provides reagents and methods for assessing tumor progression in an individual with cancer, in particular wherein said methods are used to establish tumor stage and to assess the efficacy of anticancer therapies. The invention also provides methods for providing patient individualized anticancer treatment and responses to treatment, wherein a particular treatment is administered to an individual patient based on the tumor type and stage established using the inventive methods, and the treatment maintained or changed based on the patient's individual response to said treatment.

In a first aspect, the invention provides reagents, particularly immunological reagents, and methods for assessing tumor progression, particularly colorectal cancer progression, in an individual. In this aspect, a tissue or tumor cell-containing sample from the patient is analyzed to detect a pattern of expression, phosphorylation or both expression and phosphorylation of one or more biological markers. In particular embodiments, said biological markers are members of the EGF metabolic pathway, including but not limited to EGFR, PTEN, pAKT, pMEK, pHER1, pERK, or Ki67. In these embodiments, tumor progression is assessed wherein the pattern of expression, phosphorylation or both expression or phosphorylation of one or more particularly a plurality of these biological markers is substantially similar to the pattern of expression, phosphorylation, or both expression and phosphorylation of the one or more particularly a plurality of these biological markers from a tissue or cell sample having an established stage of progression of said tumor.

In alternative embodiments, the invention provides reagents, particularly immunological reagents, and methods for assessing tumor progression, particularly colorectal cancer progression, in an individual. In this aspect, a tissue or tumor cell-containing sample from the patient is analyzed to detect a pattern of expression, phosphorylation or both expression and phosphorylation of one or more biological markers. In particular embodiments, said biological markers are members of the EGF metabolic pathway, including but not limited to EGFR, PTEN, pAKT, pMEK, pHER1, pERK, or Ki67. In these embodiments, tumor progression is assessed wherein the pattern of expression, phosphorylation or both expression or phosphorylation of one or more particularly a plurality of these biological markers differs from the pattern of expression, phosphorylation, or both expression and phosphorylation of the one or more particularly a plurality of these biological markers from a non-tumor tissue or cell sample comprising normal but not tumor cells.

Preferred uses of the reagents and methods of the invention is for assessing the clinical stage of colorectal carcinoma, wherein the biological markers comprising said pattern of expression, phosphorylation or expression and phosphorylation include EGFR, PTEN, pMEK, Ki67, and pHER1. Also falling within the scope of these aspects of the invention is the further step of measuring gene amplification of genomic DNA encoding EGFR. In certain embodiments, these assays detect balanced disomy in genomic EGFR-encoding DNA.

In these aspects, the tissue sample is preferably a small adenoma or adenomatous polyp. In particular, in these embodiments, the invention provides a pattern of expression, phosphorylation or both expression or phosphorylation wherein expression, phosphorylation or both expression or phosphorylation of EGFR is increased; expression, phosphorylation or both expression or phosphorylation of PTEN is increased; expression, phosphorylation or both expression or phosphorylation of pMEK is reduced over the levels of expression, phosphorylation or both expression or phosphorylation of these biological markers in a non-tumor tissue or cell sample.

In other embodiments, the tumor is an adenocarcinoma. In particular, in these embodiments, the invention provides a pattern of expression, phosphorylation or both expression or phosphorylation wherein expression, phosphorylation or both expression or phosphorylation of PTEN is reduced and expression, phosphorylation or both expression or phosphorylation of pMEK is increased over the levels of expression, phosphorylation or both expression or phosphorylation of these biological markers in a small adenoma tissue or cell sample. These embodiments can have within their scope the further step of measuring gene amplification of genomic DNA encoding EGFR. In certain embodiments, these assays detect balanced disomy and balanced trisomy in genomic EGFR-encoding DNA.

In still further embodiments, the tissue sample from the patient is a malignant sample, and the invention provides a pattern of expression, phosphorylation or both expression or phosphorylation wherein expression, phosphorylation or both expression or phosphorylation of PTEN is reduced and expression, phosphorylation or both expression or phosphorylation of pMEK is increased over the levels of expression, phosphorylation or both expression or phosphorylation of these biological markers in a small adenoma tissue or cell sample. These embodiments can have within their scope the further step of measuring gene amplification of genomic DNA encoding EGFR. In certain embodiments, these assays detect balanced disomy and balanced polysomy in genomic EGFR-encoding DNA.

In preferred embodiments, these patterns of expression, phosphorylation or both expression and phosphorylation of the biological markers comprising the patterns detected in the inventive methods are detected by immunohistochemistry or *in situ* hybridization. In preferred embodiments, said methods comprise assays performed advantageously using computer-aided image analysis of the tissue section following staining using a labeled specific binding reagent, preferably an immunological reagent.

In the practice of the methods of this invention are provided methods for identifying an individual bearing a tumor, particularly a colorectal cancer tumor, that is responsive to one or a combination of particular chemotherapeutic agents. In these embodiments, the inventive methods are used to detect a pattern of expression, phosphorylation or both expression or phosphorylation of one or a plurality of biological markers. In certain embodiments, the tumor is colorectal cancer and the biological markers include but are not limited to EGFR, PTEN, pAKT, pMEK, pHER1, pERK, or Ki67. In advantageous embodiments of the inventive methods, the pattern of expression, phosphorylation or both expression and phosphorylation identify the tumor as being responsive to one or a combination of chemotherapeutic agents. In specific embodiments, the inventive methods are useful for identifying tumor samples responsive to chemotherapeutic agents including but not limited to EGFR antibody or a kinase inhibitor, or both an EGFR antibody and a kinase inhibitor.

In additional aspects, the invention provides a kit for the practice of the inventive methods. In useful embodiments, said kits comprise at least two reagents, preferably specific binding agents and more preferably immunological reagents, for detecting expression, phosphorylation or both expression and phosphorylation of biological markers informative regarding tumor progression or chemotherapeutic agent responsiveness or both in a human tumor sample. In certain embodiments, the at least two reagents are useful for detecting expression, phosphorylation or both expression and phosphorylation of biological markers including but not limited to EGFR, pEGFR, PTEN, pAKT, pMEK, pHER1, pERK, or Ki67. In certain embodiments, the at least two biological markers are PTEN and pMEK. In certain embodiments, said kits comprise reagents for detecting expression, phosphorylation or both expression and phosphorylation of at least three biological markers, preferably including but not limited to EGFR, pEGFR, PTEN, pMEK, pERK, or Ki67. In certain embodiments, the at least three biological markers are EGFR, PTEN and pMEK. In alterative embodiments, the kit comprises reagents for detecting expression, phosphorylation or both expression and phosphorylation of EGFR, pEGFR, PTEN, pMEK, pERK, or Ki67. Certain embodiments of the kits of the invention also include reagents for detecting gene amplification of genomic DNA encoding EGFR. Each embodiment of said kits of the invention advantageously further comprise instructions for using the kits in the practice of the methods of the invention.

Specific preferred embodiments of the present invention will become evident from the following more detailed description of certain preferred embodiments and the claims.

### DESCRIPTION OF THE DRAWING

The patent or application file contains at least one drawing executed in color. Copies of this patent or patent application publication with color drawing(s) will be provided by the Office upon request and payment of the necessary fee.

**FIGURE 1** is a schematic diagram of the EGFR pathway.

**FIGURE 2**

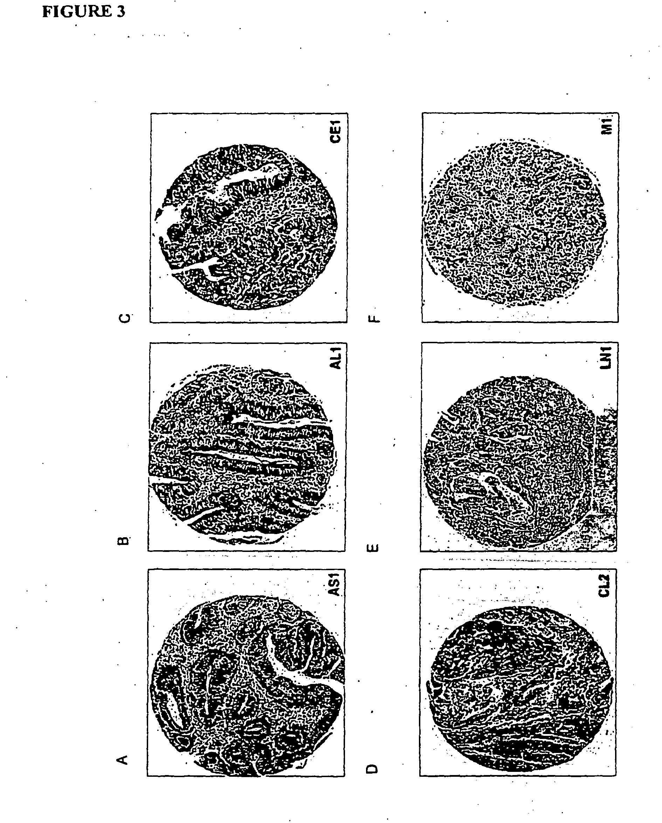

**FIGURE 3** is a photomicrograph of a representative of hematoxylin and eosin
(H&E) staining for CHTN colorectal cancer progression. Figure 3A is a representative H&E staining of an adenoma < 2cm in maximum dimension. Figure 3B is a representative H&E staining of an adenoma > 2cm in maximum dimension. Figure 3C is a representative H&E staining of a tumor sample of primary invasive pathological stage T1 or T2. Figure 3D is a representative H&E staining of a tumor sample of primary invasive pathological stage T3 or T4. Figure 3E is a representative H&E staining of a colorectal adenocarcinoma that is metastatic to the lymph nodes. Figure 3F is a representative H&E staining of a colorectal adenocarcinoma that is metastatic to distant sites.

**FIGURE 4** represents a map to the colorectal progression tissue microarray (TMA; CHTN2003CRCProg) obtained from the Cooperative Human Tissue Network (CHTN).

**FIGURE 5** represents the results of a histochemistry tissue assessment; the ptyr expression in colorectal tissue samples as assayed by IHC is represented, expressed as averaged percent positive.

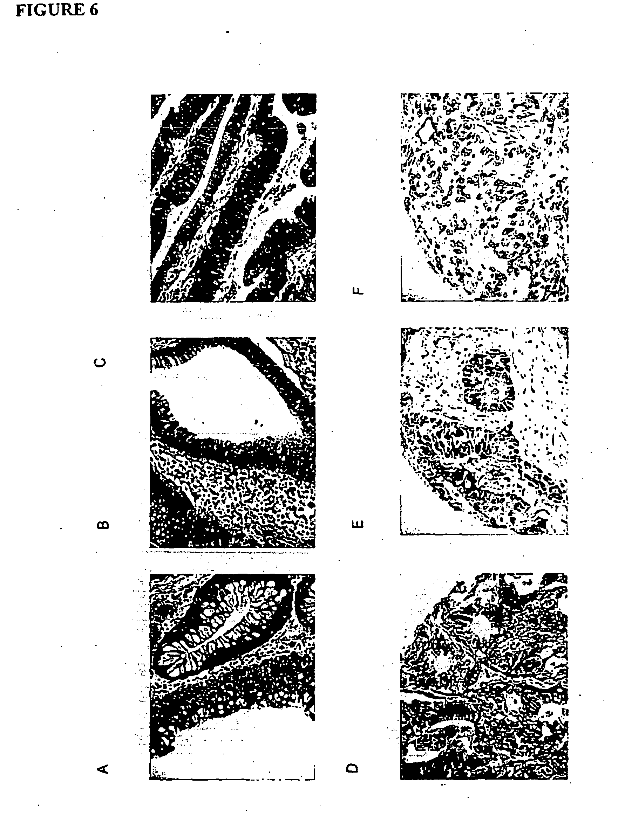

**FIGURE 6** is a photomicrograph of representative EGFR expression levels for colorectal cancer progression. Figure 6A is a representative image of EGFR staining of an adenoma < 2cm in maximum dimension (C10); the sample has a score of 3+ with 100% percent positive cytoplasmic/membrane staining. Figure 6B is a representative image of EGFR staining of an adenoma > 2cm in maximum dimension (F5); the sample has a score of 3+ with 100% percent positive cytoplasmic/membrane staining. Figure 6C is a representative image of EGFR staining of a tumor sample of primary invasive pathological stage T1 or T2 (E4); the sample has a score of 3+ with 100% percent positive cytoplasmic/membrane staining. Figure 6D is a representative image of EGFR staining of a tumor sample of primary invasive pathological stage T3 or T4 (H10); the sample has a score of 3+ with 90% percent positive cytoplasmic/membrane staining. Figure 6E is a representative image of EGFR staining of a colorectal adenocarcinoma that is metastatic to the lymph nodes (J20); the sample has a score of 2+ with 85% percent positive cytoplasmic/membrane staining. Figure 6F is a representative image of EGFR staining of a colorectal adenocarcinoma that is metastatic to distant sites (B 13); the sample has a score of 1+ with 1% percent positive membrane staining.

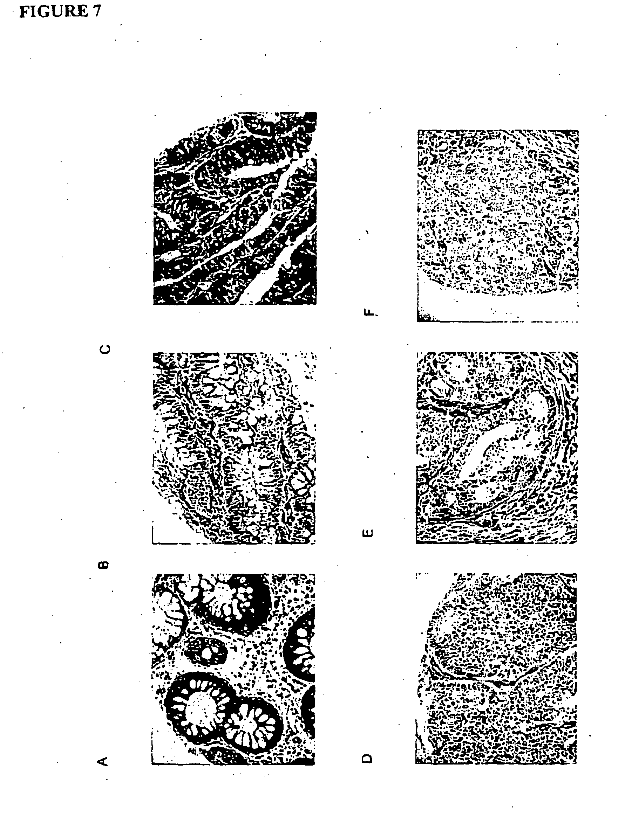

**FIGURE 7** is a photomicrograph of a representative PTEN expression levels for colorectal cancer progression. Figure 7A is a representative image of PTEN staining of an adenoma < 2cm in maximum dimension (C4); the sample has a score of 3+ with 80% percent positive cytoplasmic staining. Figure 7B is a representative image of PTEN staining of an adenoma > 2cm in maximum dimension (F3); the sample has a score of 1+ with 75% percent positive cytoplasmic staining. Figure 7C is a representative image of PTEN staining of a tumor sample of primary invasive pathological stage T1 or T2 (E2); the sample has a score of 2+ with 80% percent positive cytoplasmic staining. Figure 7D is a representative image of PTEN staining of a tumor sample of primary invasive pathological stage T3 or T4 (H4); the sample has a score of 1+ with 40% percent positive cytoplasmic staining. Figure 7E is a representative image of PTEN staining of a colorectal adenocarcinoma that is metastatic to the lymph nodes (J18); the sample was negative. Figure 7F is a representative image of PTEN staining of a colorectal adenocarcinoma that is metastatic to distant sites (B7); the sample was negative.

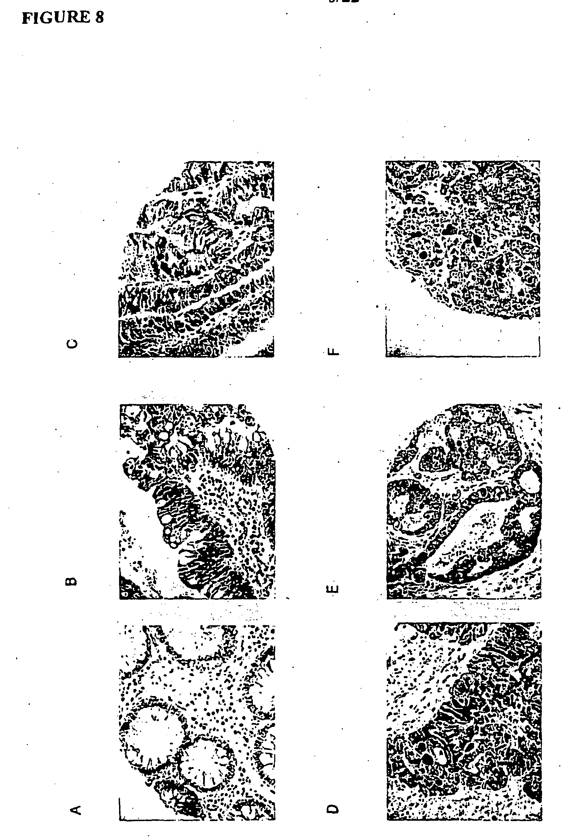

**FIGURE 8** is a photomicrograph of a representative pMEK expression levels for colorectal cancer progression. Figure 8A is a representative image of pMEK staining of an adenoma < 2cm in maximum dimension (C4); the sample has a score of 1+ with 10% percent positive cytoplasmic staining. Figure 8B is a representative image of pMEK staining of an adenoma > 2cm in maximum dimension (F3); the sample has a score of 1+ with 70% percent positive cytoplasmic staining. Figure 8C is a representative image of pMEK staining of a tumor sample of primary invasive pathological stage T1 or T2 (E2); the sample has a score of 1+ with 80% percent positive cytoplasmic staining and 1+ with 10% percent positive nuclear staining. Figure 8D is a representative image of pMEK staining of a tumor sample of primary invasive pathological stage T3 or T4 (H4); the sample has a score of 2+ with 100% percent positive cytoplasmic staining and 2+ with 5% percent positive nuclear staining. Figure 8E is a representative image of pMEK staining of a colorectal adenocarcinoma that is metastatic to the lymph nodes (J18); the sample has a score of 3+ with 100% percent positive cytoplasmic staining. Figure 8F is a representative image of pMEK staining of a colorectal adenocarcinoma that is metastatic to distant sites (B7); the sample has a score of 2+ with 100% percent positive cytoplasmic staining and 3+ with 15% percent positive nuclear staining.

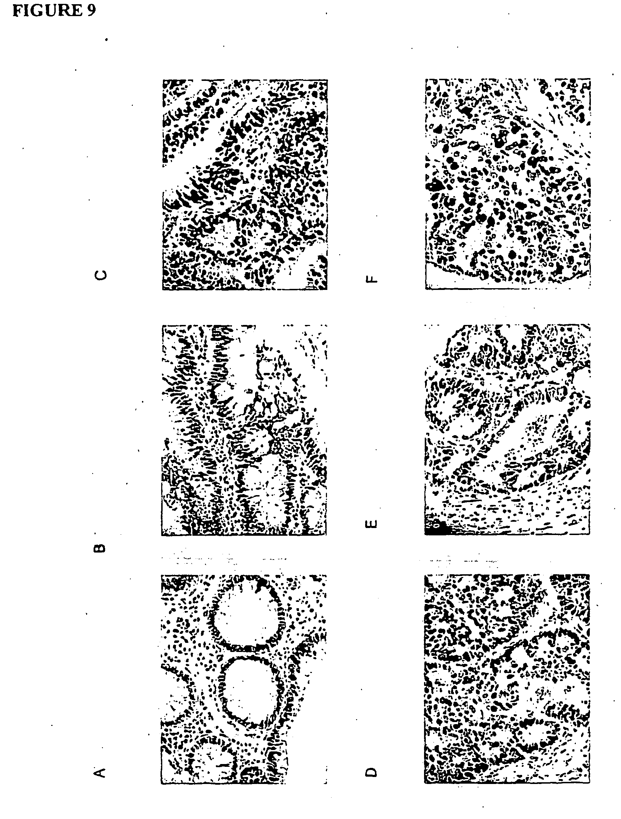

**FIGURE 9** is a photomicrograph of a representative Ki67 expression levels for colorectal cancer progression. Figure 9A is a representative image of Ki67 staining of an adenoma < 2cm in maximum dimension (C4); the sample has a score of 2+ with 10% percent positive nuclear staining. Figure 9B is a representative image of Ki67 staining of an adenoma > 2cm in maximum dimension (F3); the sample has a score of 2+ with 25% percent positive nuclear staining. Figure 9C is a representative image of Ki67 staining of a tumor sample of primary invasive pathological stage T1 or T2 (E2); the sample has a score of 2+ with 85% percent positive nuclear staining. Figure 9D is a representative image of Ki67 staining of a tumor sample of primary invasive pathological stage T3 or T4 (H4); the sample has a score of 2+ with 40% percent positive and 3+ with 15% percent positive nuclear staining. Figure 9E is a representative image of Ki67 staining of a colorectal adenocarcinoma that is metastatic to the lymph nodes (J10); the sample has a score of 3+ with 45% percent positive nuclear staining. Figure 9F is a representative image of Ki67 staining of a colorectal adenocarcinoma that is metastatic to distant sites (B7); the sample has a score of 2+ with 70% percent positive cytoplasmic staining.

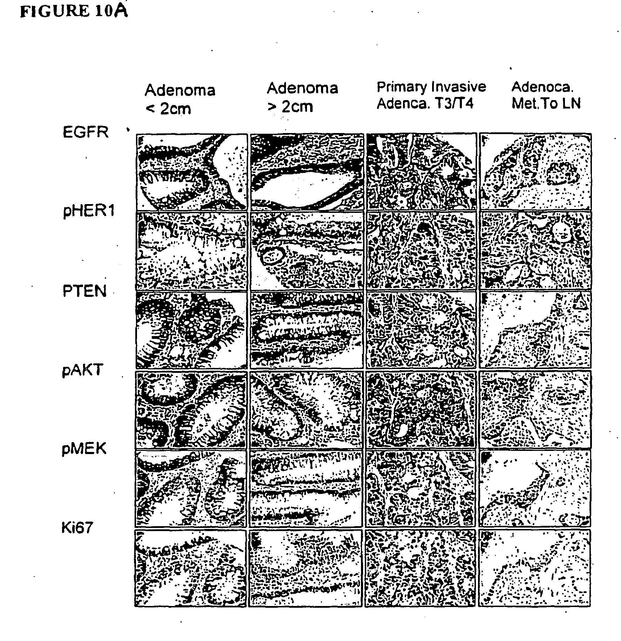

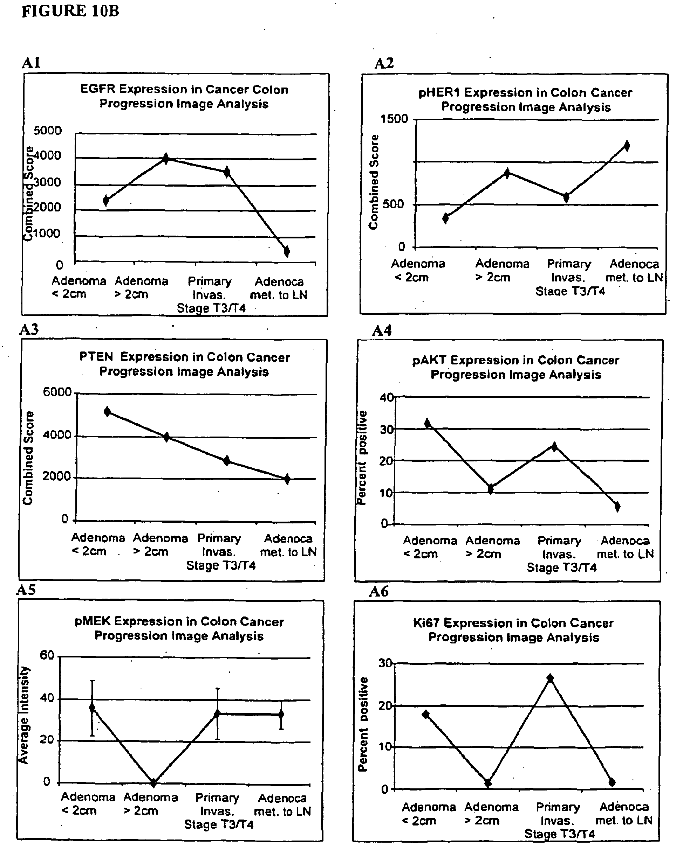

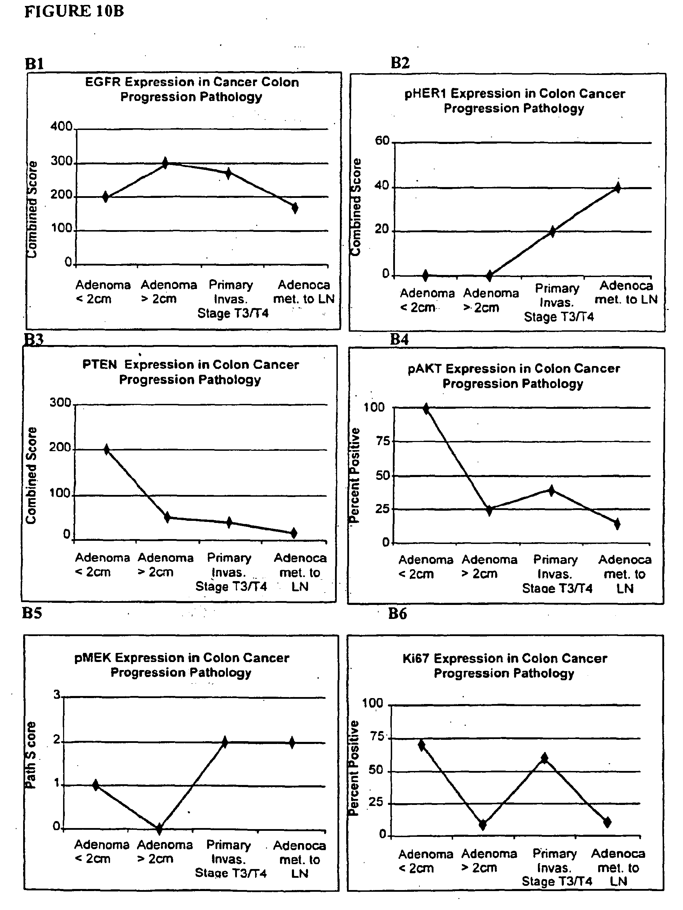

**FIGURE 10** is representative photomicrographs of biomarker expression levels of colorectal cancer progression from a single individual case (male, 71 years of age). Figure 10A are representative immunohistochemistry (IHC) images of an adenoma < 2cm in maximum dimension; an adenoma > 2cm in maximum dimension, a tumor of primary invasive pathological stage T3 or T4; and a colorectal adenocarcinoma that is metastatic to the lymph nodes. Antibodies reactive to EGFR, pHER1, PTEN, pAKT, pMEK, and Ki67 were used. Figure 10B shows the results in graphical form, both using the results of computer-aided image analysis (A), and pathology score (B).

**FIGURE 11** represents a summary of biomarker assessment in the EGFR expression pathway during colorectal cancer progression as determined using image analysis. The results are shown as % change in average score.

**FIGURE 12** is a photomicrograph of dual-color fluorescent in situ hybridization assays with probes for epidermal growth factor receptor (*EGFR,* red); chromosome 7 (CEP7, green). Figure 12A shows balanced disomy; figure 12B shows balanced trisomy, figure 12C shows balanced polysomy, and figure 12D shows gene amplification.

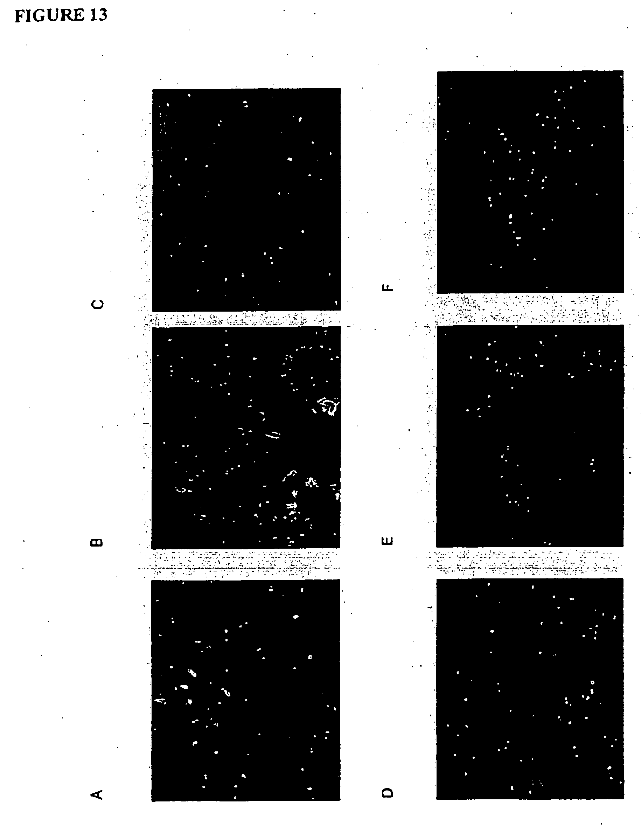

**FIGURE 13** is a photomicrograph of EGFR FISH gene detection in colorectal cancer progression. Figure 13A is a representative EGFR FISH gene detection of an adenoma < 2cm in maximum dimension, which is balanced disomy. Figure 13B is a representative EGFR FISH gene detection of an adenoma > 2cm in maximum dimension which is balanced disomy. Figure 13C is a representative EGFR FISH gene detection of a tumor sample of primary invasive pathological stage T1 or T2, which is balanced disomy. Figure 13D is a representative EGFR FISH gene detection of a tumor sample of primary invasive pathological stage T3 or T4 which is balanced polysomy. Figure 13E is a representative EGFR FISH gene detection of a colorectal adenocarcinoma that is metastatic to the lymph nodes, which is balanced polysomy. Figure 13F is a representative EGFR FISH gene detection of a colorectal adenocarcinoma that is metastatic to distant sites which is shows gene amplification disomy.

**FIGURE 14** is a photomicrograph of EGFR expression levels in colorectal cancer. Figure 14A shows two regions (Region 1 and Region 2) at a 20X scan pass; and Figure 14B shows the results for the EGFR combined score (ARIOL) for the two regions.

**FIGURE 15** is a photomicrograph of HER2 expression levels in colorectal cancer. Figure 15A shows two regions (Region 1 and Region 2) at a 20X scan pass; and Figure 15B shows the results for the HER2 combined score (ARIOL) for the two regions.

**FIGURE 16** is a photomicrograph of pAKT expression levels in colorectal cancer. Figure 16A shows two regions (Region 1 and Region 2) at a 20X scan pass; and Figure 16B shows the results for the pAKT combined score (ARIOL) for the two regions.

**FIGURE 17** is a photomicrograph of Ki67 expression levels in colorectal cancer. Figure 17A shows two regions (Region 1 and Region 2) at a 20X scan pass; and Figure 17B shows the results for the Ki67 combined score (ARIOL) for the two regions.

**FIGURE 18** is a photomicrograph of Survivin expression levels in colorectal cancer. Figure 18A shows two regions (Region 1 and Region 2) at a 20X scan pass; and Figure 18B shows the results for the Survivin combined score (ARIOL) for the two regions.

**FIGURE 19** is a photomicrograph of VEGF expression levels in colorectal cancer. Figure 19A shows two regions (Region 1 and Region 2) at a 20X scan pass; and Figure 19B shows the results for the VEGF combined score (ARIOL) for the two regions.

**FIGURE 20** shows a schematic diagram of silver in situ hybridization.

### DETAILED DESCRIPTION OF PREFERRED EMBODIMENTS

This invention provides methods for assessing colorectal cancer progression in individuals, including cancer patients. In addition, this invention provides predictive biomarkers for assessing colorectal cancer progression. Furthermore, this invention provides methods for identifying a colorectal cancer tumor responsive to a chemotherapeutic agent. Moreover, this invention provides kits for assessing colorectal cancer progression.

In contrast to traditional anticancer methods, where chemotherapeutic drug treatment is undertaken as an adjunct to and after surgical intervention, neoadjuvant (or primary) chemotherapy consists of administering drugs as an initial treatment in certain cancer patients. One advantage of such an approach is that, for primary tumors of more than 3 cm, it permits the later or concomitant use of conservative surgical procedures (as opposed to, *e.g*., radical mastectomy in breast cancer patients) for the majority of patients, due to the tumor shrinking effect of the chemotherapy. Another advantage is that for many cancers, a partial and/or complete response is achieved in about two-thirds of all patients. Finally, because the majority of patients are responsive after two to three cycles of chemotherapeutic treatment, it is possible to monitor the *in vivo* efficacy of the chemotherapeutic regimen employed, in order to identify patients whose tumors are non-responsive to chemotherapeutic treatment. Timely identification of non-responsive tumors allows the clinician to limit a cancer patient's exposure to unnecessary side-effects of treatment and to institute alternative treatments. Unfortunately, methods present in the art, including histological examination, are insufficient for optimum application of such timely and accurate identification. The present invention provides methods for developing more informed and effective regimes of therapy that can be administered to cancer patients with an increased likelihood of an effective outcome (*i.e.*, reduction or elimination of the tumor).

A cancer diagnosis, both an initial diagnosis of disease and subsequent monitoring of the disease course (before, during, or after treatment) is conventionally confirmed through histological examination of cell or tissue samples removed from a patient. Clinical pathologists need to be able to accurately determine whether such samples are benign or malignant and to classify the aggressiveness of tumor samples deemed to be malignant, because these determinations often form the basis for selecting a suitable course of patient treatment. Similarly, the pathologist needs to be able to detect the extent to which a cancer has grown or gone into remission, particularly as a result of or consequent to treatment, most particularly treatment with chemotherapeutic or biological agents.

Histological examination traditionally entails tissue-staining procedures that permit morphological features of a sample to be readily observed under a light microscope. A pathologist, after examining the stained sample, typically makes a qualitative determination of whether the tumor sample is malignant. It is difficult, however, to ascertain a tumor's aggressiveness merely through histological examination of the sample, because a tumor's aggressiveness is often a result of the biochemistry of the cells within the tumor, such as protein expression or suppression and protein phosphorylation, which may or may not be reflected by the morphology of the sample. Therefore, it is important to be able to assess the biochemistry of the cells within a tumor sample. Further, it is desirable to be able to observe and quantitate both gene expression and protein phosphorylation of tumor-related genes or proteins, or more specifically cellular components of tumor-related signaling pathways.

Cancer therapy can be based on molecular profiling of tumors rather than simply their histology or site of the disease. Elucidating the biological effects of targeted therapies in tumor tissue and correlating these effects with clinical response helps identify the predominant growth and survival pathways operative in tumors, thereby establishing a pattern of likely responders and conversely providing a rational for designing strategies to overcome resistance. For example, successful diagnostic targeting of a growth factor receptor must determine if tumor growth or survival is being driven by the targeted receptor or receptor family, by other receptors not targeted by the therapy, and whether downstream signaling suggests that another oncogenic pathway is involved. Furthermore, where more than one signaling pathway is implicated, members of those signaling pathways can be used as diagnostic targets to determine if a dual inhibitor therapy will be or is effective.

In order for chemotherapy to be effective, the medications should destroy tumor cells and spare the normal body cells, particularly those normal cells that may be adjacent or in proximity to the tumor. This can be accomplished, *inter alia,* by using medications that affect cell activities that go on predominantly in cancer cells but not in normal cells.

Automated (computer-aided) image analysis systems known in the art can augment visual examination of tumor samples. In a representative embodiment, the cell or tissue sample is exposed to detectably-labeled reagents specific for a particular biological marker, and the magnified image of the cell is then processed by a computer that receives the image from a charge-coupled device (CCD) or camera such as a television camera. Such a system can be used, for example, to detect and measure expression and activation levels of EGFR, ptyr, PTEN, pAKT, pMEK, pHER1, pERK, or KI67 in a sample, or any additional diagnostic biomarkers. Thus, the methods of the invention provide more accurate cancer diagnosis and better characterization of gene expression in histologically identified cancer cells, most particularly with regard to expression of tumor marker genes or genes known to be expressed in particular cancer types and subtypes (*e.g*., having different degrees of malignancy). This information permits a more informed and effective regimen of therapy to be administered, because drugs with clinical efficacy for certain tumor types or subtypes can be administered to patients whose cells are so identified.

Another drawback of conventional anticancer therapies is that the efficacy of specific chemotherapeutic agents in treating a particular cancer in an individual human patient is unpredictable. In view of this unpredictability, the art is unable to determine, prior to starting therapy, whether one or more selected agents would be active as antitumor agents or to render an accurate prognosis or course of treatment in an individual patient. This is especially important because a particular clinical cancer may present the clinician with a choice of treatment regimens, without any current way of assessing which regimen will be most efficacious for a particular individual. It is an advantage of the methods of this invention that they are able to better assess the expected efficacy of a proposed therapeutic agent (or combination of agents) in an individual patient. The claimed methods are advantageous for the additional reasons that they are both time-and cost-effective in assessing the efficacy of chemotherapeutic regimens and are minimally traumatic to cancer patients.

Patterns of expression and phosphorylation of polypeptides are detected and quantified using methods of the present invention. More particularly, patterns of expression and phosphorylation of polypeptides that are cellular components of a tumor-related signaling pathway are detected and quantified using methods of the present invention. For example, the patterns of expression and phosphorylation of polypeptides can be detected using biodetection reagents specific for the polypeptides, including but not limited to antibodies. Alternatively, the biodetection reagents can be nucleic acid probes.

As used with the inventive methods disclosed herein, a nucleic acid probe is defined to be a collection of one or more nucleic acid fragments whose hybridization to a sample can be detected. The probe may be unlabeled or labeled so that its binding to the target or sample can be detected. The probe is produced from a source of nucleic acids from one or more particular (preselected) portions of the genome, *e.g*., one or more clones, an isolated whole chromosome or chromosome fragment, or a collection of polymerase chain reaction (PCR) amplification products. The nucleic acid probe may also be isolated nucleic acids immobilized on a solid surface (*e.g*., nitrocellulose, glass, quartz, fused silica slides), as in an array. The probe may be a member of an array of nucleic acids as described, for instance, in WO 96/17958. Techniques capable of producing high density arrays can also be used for this purpose *(see, e.g.,* Fodor, 1991, Science X: 767-773; Johnston, 1998, Curr. Biol. 8: R171-R174; Schummer, 1997, Biotechniques 23: 1087-1092; Kern, 1997, Biotechniques 23: 120-124; U.S. Pat. No. 5,143,854). One of skill will recognize that the precise sequence of the particular probes can be modified to a certain degree to produce probes that are "substantially identical," but retain the ability to specifically bind to (*i.e.,* hybridize specifically to) the same targets or samples as the probe from which they were derived. The term "nucleic acid" refers to a deoxyribonucleotide or ribonucleotide in either single- or doublestranded form. The term encompasses nucleic acids, *e.g*., oligonucleotides, containing known analogues of natural nucleotides that have similar or improved binding properties, for the purposes desired, as the reference nucleic acid. The term also includes nucleic acids which are metabolized in a manner similar to naturally occurring nucleotides or at rates that are improved for the purposes desired. The term also encompasses nucleic-acid-like structures with synthetic backbones. One of skill in the art would recognize how to use a nucleic acid probes for screening of cancer cells in a sample by reference, for example, to U.S. Patent 6,326,148, directed to screening of colon carcinoma cells.

Polypeptides associated with cancer can be quantified by image analysis using a suitable primary antibody against biomarkers, including but not limited EGFR, PTEN, pAKT, pMEK, p, pERK, or Ki67, detected directly or using an appropriate secondary antibody (such as rabbit anti-mouse IgG when using mouse primary antibodies) and/or a tertiary avidin (or Strepavidin) biotin complex ("ABC").

Examples of reagents useful in the practice of the methods of the invention as exemplified herein include immunological reagents. By "immunological reagent" is meant antibodies, including particularly polyclonal antisera and monoclonal antibodies. Antibodies of the invention can be produced by any method known in the art for the synthesis of antibodies, including chemical synthesis or recombinant expression techniques, or preferably using conventional immunological methods. As used herein, the term "antibody" includes, but is not limited to, both naturally occurring and non-naturally occurring antibodies. As used herein, the term "antibody" is intended to refer broadly to any immunologic binding agent such as IgG, IgM, IgA, IgD and IgE. Generally, IgG and/or IgM are preferred because they are the most common antibodies in the physiological situation and because they are most easily made in a laboratory setting. More specifically, the term "antibody" includes polyclonal and monoclonal antibodies, and antigen-binding fragments thereof such as Fab, Fab', and F(ab')₂ fragments. Furthermore, the term "antibody" includes chimeric antibodies and wholly synthetic antibodies, including genetically engineered antibodies, and fragments thereof. The polyclonal and monoclonal antibodies may be "purified" which means the polyclonal and monoclonal antibodies are free of any other antibodies.

Methods for preparing polyclonal and monoclonal antibodies are well known in the art (*see for example,* Sambrook et al., 1989, MOLECULAR CLONING: A LABORATORY MANUAL, Second Edition, Cold Spring Harbor, N.Y.; and Hurrell (Ed.), MONOCLONAL HYBRIDOMA ANTIBODIES: TECHNIQUES AND APPLICATIONS, CRC Press, Inc., Boca Raton, Fla., which are incorporated herein by reference). As would be evident to one of ordinary skill in the art, polyclonal antibodies can be generated from a variety of warm-blooded animals such as horses, cows, goats, sheep, dogs, chickens, rabbits, mice, and rats. The immunogenicity of an antigenic epitope can be increased through the use of an adjuvant such as Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum. Such adjuvants are also well known in the art. Information concerning adjuvants and various aspects of immunoassays are disclosed, for example, in Tijssen (1987, PRACTICE AND THEORY OF ENZYME IMMUNOASSAYS, 3rd Ed., Elsevier: New York). Other useful references covering methods for preparing polyclonal antisera include MICROBIOLOGY (1969, Hoeber Medical Division, Harper and Row); Landsteiner (1962, SPECIFICITY OF SEROLOGICAL REACTIONS, Dover Publications: New York), and Williams et al. (1967, METHODS IN IMMUNOLOGY AND IMMUNOCHEMISTRY, Vol. 1, Academic Press: New York).

The amount of immunogen composition used in the production of polyclonal antibodies varies upon the nature of the immunogen as well as the animal used for immunization. A variety of routes can be used to administer the immunogen (subcutaneous, intramuscular, intradermal, intravenous and intraperitoneal). The production of polyclonal antibodies may be monitored by sampling blood of the immunized animal at various points following immunization. A second, booster, injection may also be given. The process of boosting and titering is repeated until a suitable titer is achieved. When a desired level of immunogenicity is obtained, the immunized animal can be bled and the serum isolated and stored.

Serum produced from animals immunized using standard methods can be used directly, or the IgG fraction can be separated from the serum using standard methods such as plasmaphoresis or adsorption chromatography with IgG-specific adsorbents such as immobilized Protein A.

Antibody fragments, such F(ab')₂ and Fab fragments, can be produced from the corresponding antibodies by cleavage of and collection of the desired fragments in accordance with known methods (*see, for example,* Andrew et al., 1992, "Fragmentation of Immunoglobulins" in CURRENT PROTOCOLS IN IMMUNOLOGY, Unit 2.8, Greene Publishing Assoc. and John Wiley & Sons).

Alternatively, monoclonal antibodies against the antigenic peptides of the invention can be prepared according to well-known techniques, such as those exemplified in U.S. Pat. No. 4,196,265, incorporated herein by reference. Hybridomas producing monoclonal antibodies against the antigenic peptides of the invention are produced by well-known techniques. Usually, the process involves the fusion of an immortalizing cell line with a B-lymphocyte that produces the desired antibody. Immortalizing cell lines are usually transformed mammalian cells, particularly myeloma cells of rodent, bovine, and human origin. Rodents such as mice and rats are preferred animals, however, the use of rabbit or sheep cells is also possible. Mice are preferred, with the BALB/c mouse being most preferred as this is most routinely used and generally gives a higher percentage of stable fusions.

Techniques for obtaining antibody-producing lymphocytes from mammals injected with antigens are well known. Generally, peripheral blood lymphocytes (PBLs) are used if cells of human origin are employed, or spleen or lymph node cells are used from non-human mammalian sources. A host animal is injected with repeated dosages of the purified antigen, and the animal is permitted to generate the desired antibody-producing cells before they are harvested for fusion with the immortalizing cell line. Most frequently, immortalized cell lines are rat or mouse myeloma cell lines that are employed as a matter of convenience and availability. Techniques for fusion are also well known in the art, and in general involve mixing the cells with a fusing agent, such as polyethylene glycol.

Generally, following immunization somatic cells with the potential for producing antibodies, specifically B-lymphocytes (B-cells), are selected for use in the mAb generating protocol. These cells may be obtained from biopsied spleens, tonsils or lymph nodes, or from a peripheral blood sample. Spleen cells and peripheral blood cells are preferred, the former because they are a rich source of antibody-producing cells that are in the dividing plasmablast stage, and the latter because peripheral blood is easily accessible. Often, a panel of animals will have been immunized and the spleen of animal with the highest antibody titer will be removed and the spleen lymphocytes obtained by homogenizing the spleen with a syringe. Typically, a spleen from an immunized mouse contains approximately fifty million to two hundred million lymphocytes.

Myeloma cell lines suited for use in hybridoma-producing fusion procedures preferably are non-antibody-producing, have high fusion efficiency, and enzyme deficiencies that render then incapable of growing in certain selective media which support the growth of only the desired fused cells (hybridomas). Any one of a number of myeloma cells may be used, as are known to those of skill in the art. Available murine myeloma lines, such as those from the American Type Culture Collection (ATCC), 10801 University Boulevard, Manassas, Va. 20110-2209, USA, may be used in the hybridization. For example, where the immunized animal is a mouse, one may use P3-X63/Ag8, X63-Ag8.653, NS1/1.Ag 4 1, Sp210-Ag14, FO, NSO/U, MPC-11, MPC11-X45-GTG 1.7 and S194/5XX0 Bul; for rats, one may use R210.RCY3, Y3-Ag 1.2.3, 1R983F and 4B210; and U-266, GM1500-GRG2, LICR-LON-HMy2 and UC729-6 are all useful in connection with human cell fusions. One preferred murine myeloma cell is the NS-1 myeloma cell line (also termed P3-NS-1-Ag4-1), which is readily available from the NIGMS Human Genetic Mutant Cell Repository by requesting cell line repository number GM3573. Another mouse myeloma cell line that may be used is the 8-azaguanine-resistant mouse murine myeloma SP2/0 non-producer cell line.

Methods for generating hybrids of antibody-producing spleen or lymph node cells and myeloma cells usually comprise mixing somatic cells with myeloma cells in a 2:1 ratio, though the ratio may vary from about 20:1 to about 1:1, respectively, in the presence of an agent or agents (chemical or electrical) that promote the fusion of cell membranes. Fusion methods using Sendai virus have been described (Kohler et al., 1975, Nature 256:495; Kohler et al., 1976, Eur. J. Immunol. 6:511; Kohler et al., 1976, Eur. J. Immunol. 6 :292), and those using polyethylene glycol (PEG), such as 37% (v/v) PEG, by Gefter et al. (1977, Somatic Cell Genet 3: 231-236). The use of electrically induced fusion methods is also appropriate (Goding, 1986).

Fusion procedures usually produce viable hybrids at low frequencies, about 1 x 10⁻⁶ to 1 x 10⁻⁸. However, this does not pose a problem, as the viable, fused hybrids are differentiated from the parental, unfused cells (particularly the unfused myeloma cells that would normally continue to divide indefinitely) by culturing in a selective medium. The selective medium is generally one that contains an agent that blocks the de novo synthesis of nucleotides in the tissue culture media. Exemplary and preferred agents are aminopterin, methotrexate, and azaserine. Aminopterin and methotrexate block *de novo* synthesis of both purines and pyrimidines, whereas azaserine blocks only purine synthesis. Where aminopterin or methotrexate is used, the media is supplemented with hypoxanthine and thymidine as a source of nucleotides (HAT medium). Where azaserine is used, the media is supplemented with hypoxanthine. The preferred selection medium is HAT. The myeloma cells are defective in key enzymes of the salvage pathway, *e.g*., hypoxanthine phosphoribosyl transferase (HPRT), and they cannot survive. The B-cells can operate this pathway, but they have a limited life span in culture and generally die within about two weeks. Therefore, the only cells that can survive in the selective media are those hybrids formed from myeloma and B-cells.

Culturing the fusion products under these conditions provides a population of hybridomas from which specific hybridomas are selected. Typically, selection of hybridomas is performed by culturing the cells by single-clone dilution in microtiter plates, followed by testing the individual clonal supernatants (after about two to three weeks) for the desired reactivity. Hybridomas secreting the desired antibody are selected using standard immunoassays, such as Western blotting, ELISA (enzyme-linked immunosorbent assay), RIA (radioimmunoassay), or the like. Antibodies are recovered from the medium using standard protein purification techniques (such as Tijssen, 1985, *Id.).* The assay should be sensitive, simple and rapid, such as radioimmunoassay, enzyme immunoassays, cytotoxicity assays, plaque assays, dot immunobinding assays, and the like.

The selected hybridomas are then serially diluted and cloned into individual antibody-producing cell lines, which clones can then be propagated indefinitely to provide mAbs. The cell lines may be exploited for mAb production in at least two ways. A sample of the hybridoma can be injected (often into the peritoneal cavity) into a histocompatible animal of the type that was used to provide the somatic and myeloma cells for the original fusion. The injected animal develops tumors secreting the specific monoclonal antibody produced by the fused cell hybrid. The body fluids of the animal, such as serum or ascites fluid, can then be tapped to provide mAbs in high concentration. The individual cell lines could also be cultured *in vitro,* where the mAbs are naturally secreted into the culture medium from which they can be readily obtained in high concentrations. mAbs produced by either means may be further purified, if desired, using filtration, centrifugation and various chromatographic methods such as HPLC or affinity chromatography.

Many references are available to provide guidance in applying the above techniques, including Kohler et al. (1980, HYBRIDOMA TECHNIQUES, Cold Spring Harbor Laboratory, New York); Tijssen (1985, Id.); Campbell (1984, MONOCLONAL ANTIBODY TECHNOLOGY, Elsevier: Amsterdam); Hurrell (1982, *Id*.). Monoclonal antibodies can also be produced using well known phage library systems. *See, for example,* Huse et al. (1989, Science 246:1275); Ward et al. (1989, Nature 341:544).

Antibody fragments, such F(ab')₂ and Fab fragments, can be produced from the corresponding antibodies by cleavage of and collection of the desired fragments in accordance with known methods (*see, for example,* Andrew et al., 1992, "Fragmentation of Immunoglobulins" in CURRENT PROTOCOLS IN IMMUNOLOGY, Unit 2.8, Greene Publishing Assoc. and John Wiley & Sons).

Antibodies thus produced, whether polyclonal or monoclonal, can be used, *e.g*., in an immobilized form bound to a solid support by well known methods.

Antibodies can also be used, unlabeled or labeled by standard methods, as the basis for immunoassays and immunospecific binding assays. The immunoassays which can be used include but are not limited to competitive and non-competitive assay systems using techniques such as Western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (*see, e.g.,* Ausubel *et al.,* eds, 1994, *Id*.).

In particular, the antibodies of the present invention may also be used in conjunction with both fresh-frozen and/or formalin-fixed, paraffin-embedded tissue blocks prepared for study by immunohistochemistry (IHC).

Detection can be facilitated by coupling the antibody to a detectable substance. Examples of detectable substances include various enzymes, prosthetic groups, fluorescent materials, luminescent materials, bioluminescent materials, radioactive materials, positron emitting metals using various positron emission tomographies, and nonradioactive paramagnetic metal ions. The detectable substance may be coupled or conjugated either directly to the antibody (or fragment thereof) or indirectly, through an intermediate (such as, for example, a linker known in the art) using techniques known in the art. *See, for example,* U.S. Pat. No. 4,741,900 for metal ions that can be conjugated to antibodies for use as diagnostics according to the present invention. The particular label used will depend upon the type of immunoassay. Examples of labels that can be used include but are not limited to radiolabels such as ³H, ¹⁴C, ³²P, ¹²⁵I, ¹³¹I, ¹¹¹In or ⁹⁹Tc; fluorescent labels such as fluorescein and its derivatives, rhodamine and its derivatives, dansyl and umbelliferone; chemiluminescers such as luciferase and 2,3-dihydro-phthalazinediones; and enzymes such as horseradish peroxidase, alkaline phosphatase, lysozyme, glucose-6-phosphate dehydrogenase, and acetylcholinesterase. The antibodies can be tagged with such labels by known methods. For example, coupling agents such as aldehydes, carbodiimides, dimaleimide, imidates, succinimides, bisdiazotized benzadine and the like may be used to tag the antibodies with fluorescent, chemiluminescent or enzyme labels. The general methods involved are well known in the art and are described, for example, in IMMUNOASSAY: A PRACTICAL GUIDE (1987, Chan (Ed.), Academic Press, Inc.:Orlando, FL).

Further, the pattern of expression, phosphorylation, or both expression and phosphorylation of the predictive polypeptides can be compared to a non-tumor tissue or cell sample. The non-tumor tissue or cell sample can be obtained from a non-tumor tissue or cell sample from the same individual, or alternatively, a non-tumor tissue or cell sample from a different individual. A detected pattern for a polypeptide is referred to as decreased in the mammalian tumor, tissue, or cell sample, if there is less polypeptide detected as compared to the a non-tumor tissue or cell sample. A detected pattern for a polypeptide is referred to as "increased" in the mammalian tumor, tissue, or cell sample, if there is more polypeptide detected as compared to the a non-tumor tissue or cell sample. A detected pattern for a polypeptide is referred to as "normal" in the mammalian tumor, tissue, or cell sample, if there is the same, or approximately the same, polypeptide detected as compared to a non-tumor tissue or cell sample.

In practicing the methods of this invention, staining procedures can be carried out by a person, such as a histotechnician in an anatomic pathology laboratory. Alternatively, the staining procedures can be carried out using automated systems, such as Ventana Medical Systems' Benchmark® series of automated stainers. In either case, staining procedures for use according to the methods of this invention are performed according to standard techniques and protocols well-established in the art.

By "cell or tissue sample" is meant biological samples comprising cells, most preferably tumor cells, that are isolated from body samples, such as, but not limited to, smears, sputum, biopsies, secretions, cerebrospinal fluid, bile, blood, lymph fluid, urine and feces, or tissue which has been removed from organs, such as breast, lung, intestine, skin, cervix, prostate, and stomach. For example, a tissue samples can comprise a region of functionally related cells or adjacent cells.

The amount of target protein may be quantified by measuring the average optical density of the stained antigens. Concomitantly, the proportion or percentage of total tissue area stained can be readily calculated, for example as the area stained above a control level (such as an antibody threshold level) in the second image. Following visualization of nuclei containing biomarkers, the percentage or amount of such cells in tissue derived from patients after treatment are compared to the percentage or amount of such cells in untreated tissue. For purposes of the invention, "determining" a pattern of expression, phosphorylation, or both expression and phosphorylation of polypeptides is understood broadly to mean merely obtaining the expression level information on such polypeptide(s), either through direct examination or indirectly from, for example, a contract diagnostic service.

Alternatively, the amount of target protein can be determined using fluorescent methods. For example, Quantum dots (Qdots) are becoming increasingly useful in a growing list of applications including immunohistochemistry, flow cytometry, and plate-based assays, and may therefore be used in conjunction with this invention. Qdot nanocrystals have unique optical properties including an extremely bright signal for sensitivity and quantitation; high photostability for imaging and analysis. A single excitation source is needed, and a growing range of conjugates makes them useful in a wide range of cell-based applications. Qdot Bioconjugates are characterized by quantum yields comparable to the brightest traditional dyes available. Additionally, these quantum dot-based fluorophores absorb 10-1000 times more light than traditional dyes. The emission from the underlying Qdot quantum dots is narrow and symmetric which means overlap with other colors is minimized, resulting in minimal bleed through into adjacent detection channels and attenuated crosstalk, in spite of the fact that many more colors can be used simultaneously. Standard fluorescence microscopes are an inexpensive tool for the detection of Qdot Bioconjugates. Since Qdot conjugates are virtually photo-stable, time can be taken with the microscope to find regions of interest and adequately focus on the samples. Qdot conjugates are useful any time bright photo-stable emission is required and are particularly useful in multicolor applications where only one excitation source/filter is available and minimal crosstalk among the colors is required. For example, Quantum dots have been used as conjugates of Streptavidin and IgG to label cell surface markers and nuclear antigens and to stain microtubules and actin (Wu et al. 2003, Nature Biotech., 21, 41-46).

For example, QDOT Fluorescent IHC can be performed with secondary antibodies, where the detection substrates are streptavidin-conjugated Qdots (Ventana Medical Systems, Inc., Tucson, AZ) ("Ventana"). Image analysis can be performed by initially capturing image cubes on a spectral imaging camera (Cambridge Research Instruments, Woburn, MA). Excitation can be conducted with a UV (mercury) light source. The image cubes can then analyzed on a Ventana Research Imaging Application. Briefly, image cubes can be retrieved in the application and data can be extracted and reported based on the pixel intensities of Qdots expected to emit at 605nm and 655nm.

As an example, fluorescence can be measured with the multispectral imaging system Nuance^{™} (Cambridge Research & Instrumentation, Woburn, MA). As another example, fluorescence can be measured with the spectral imaging system SpectrView^{™} (Applied Spectral Imaging, Vista, CA). Multispectral imaging is a technique in which spectroscopic information at each pixel of an image is gathered and the resulting data analyzed with spectral image-processing software. For example, the Nuance system can take a series of images at different wavelengths that are electronically and continuously selectable and then utilized with an analysis program designed for handling such data. The Nuance system is able to obtain quantitative information from multiple dyes simultaneously, even when the spectra of the dyes are highly overlapping or when they are co-localized, or occurring at the same point in the sample, provided that the spectral curves are different. Many biological materials autofluoresce, or emit lower-energy light when excited by higher-energy light. This signal can result in lower contrast images and data. High-sensitivity cameras without multispectral imaging capability only increase the autofluorescence signal along with the fluorescence signal. Multispectral imaging can unmix, or separate out, autofluorescence from tissue and, thereby, increase the achievable signal-to-noise ratio.

In reference to antibody detection methods, "detection reagents" are meant reagents that can be used to detect antibodies, including both primary or secondary antibodies. For example, detection reagents can be fluorescent detection reagents, Qdots, chromogenic detection reagents, or polymer based detection systems. However, the methods and kits of the invention are not limited by these detection reagents, nor are they limited to a primary and secondary antibody scheme (for example, tertiary, etc. antibodies are contemplated by the methods of the invention).

The present invention may also use nucleic acid probes as a means of indirectly detecting the expressed protein biomarkers. For example, probes for the EGFR biomarker can be constructed using standard probe design methodology, well-know to one of ordinary skill in the probe design art. As an example, U.S. Patent application no. US20050137389A1, "Methods and compositions for chromosome-specific staining," incorporated by reference herein, describes methods of designing repeat-free probe compositions comprising heterogeneous mixtures of sequences designed to label an entire chromosome.

Gene-specific probes may be designed according to any of the following published procedures. To this end it is important to produce pure, or homogeneous, probes to minimize hybridizations at locations other than at the site of interest (Henderson, 1982, International Review of Cytology 76: 1-46). Manuelidis et al. (1984, Chromosoma 91: 28-38) discloses the construction of a single kind of DNA probe for detecting multiple loci on chromosomes corresponding to members of a family of repeated DNA sequences.

Wallace et al. (1981, Nucleic Acids Research 9:879-94) discloses the construction of synthetic oligonucleotide probes having mixed base sequences for detecting a single locus corresponding to a structural gene. The mixture of base sequences was determined by considering all possible nucleotide sequences that could code for a selected sequence of amino acids in the protein to which the structural gene corresponded.

Olsen et al. (1980, Biochemistry 19:2419-28) discloses a method for isolating labeled unique sequence human X chromosomal DNA by successive hybridizations: first, total genomic human DNA against itself so that a unique sequence DNA fraction can be isolated; second, the isolated unique sequence human DNA fraction against mouse DNA so that homologous mouse/human sequences are removed; and finally, the unique sequence human DNA not homologous to mouse against the total genomic DNA of a human/mouse hybrid whose only human chromosome is chromosome X, so that a fraction of unique sequence X chromosomal DNA is isolated.

Labeled nucleic-acid probes can be used in the methods of this invention in gene detection protocols. For example, fluorescent *in situ* hybridization ("FISH") gene detection methods can be used to determine the gene status of genes, such as the EGFR gene. FISH gene detection can be used to measure amplification, deletion, or rearrangement of genomic DNA encoding, for example, EGFR. FISH gene detection, which allows measurement of amplification, deletion, or rearrangement of genomic DNA, thereby allows detection of the gene status, for example, if the genes are balanced disomy, balanced trisomy, or balanced polysomy.

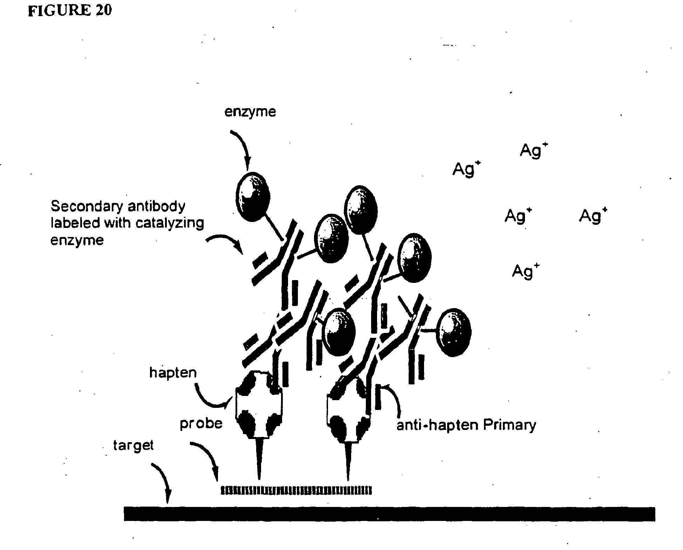

The present invention may also use methods that include silver *in situ* hybridization. With this technique, enzymes such as horseradish peroxidase (HRP) catalyze the reduction of silver ions to metallic silver; and metal particles deposit at the site of the target hybridized to the probe (Hoff et al., 2002, Am J Clin Pathol. 117: 916-21*.; see* **FIGURE 20**). For example, silver *in situ* hybridization can be used to measure amplification, deletion, and rearrangement of genomic DNA.

Cancer tissue sections taken from patients are analyzed, according to the methods of this invention by immunohistochemistry for expression, phosphorylation, or expression and phosphorylation of members of the EGF pathway or any positive treatment response predictive combination thereof. In the methods of the invention, a change in "expression" can mean a change in number of cells in which the biomarker is detected, or alternatively, the number of positive cells may be the same, but the intensity (or level) may be altered. The term expression can be used as a surrogate term indicating changes in levels of molecular activation level.

These measurements can be accomplished, for example, by using tissue microarrays. Tissue microarrays are advantageously used in the methods of the invention, being well-validated method to rapidly screen multiple tissue samples under uniform staining and scoring conditions. (Hoos et al., 2001, Am J Pathol. 158: 1245-51). Scoring of the stained arrays can be accomplished manually using the standard 0 to 3+ scale, or by an automated system that accurately quantifies the staining observed. The results of this analysis identify biomarkers that best predict patient outcome following treatment. Patient "probability of response" ranging from 0 to 100 percent can be predicted based upon the expression, phosphorylation or both of a small set of ligands, receptors, signaling proteins or predictive combinations thereof. Additional samples from cancer patients can be analyzed, either as an alternative to or in addition to tissue microarray results. For example, analysis of samples from breast cancer patients can confirm the conclusions from the tissue arrays, if the patient's responses correlate with a specific pattern of receptor expression and/or downstream signaling.

The invention provides, in part, kits for carrying out the methods of the invention. For example, the method provides kits for assessing colorectal cancer progression in an individual, comprising at least two reagents, preferably antibodies, that can detect the expression, phosphorylation, or both of polypeptides in the EGF pathway. For example, the kit can contain at least two, three, or four reagents that bind to EGFR, PTEN, pAKT, pMEK, pHER1, pERK, or Ki67. Further, the kit can include additional components other then the above-identified reagents, including but not limited to additional antibodies. Such kits may be used, for example, by a clinician or physician as an aid to selecting an appropriate therapy for a particular patient.

The Examples that follow are illustrative of specific embodiments of the invention, and various uses thereof. They set forth for explanatory purposes only, and are not to be taken as limiting the invention.

### Example 1

### Immunohistochemical staining of downstream molecules in EGF pathway in colorectal tumor progression

In order to determine whether biomarker profiles could be identified for colorectal cancer that correlate with pathology staging of carcinomas, the expression levels of biomarkers linked to the expression of EGFR in colorectal cancer cases was examined using a commercially available tissue array and tissue samples from individual cases. The biomarkers were assessed using immunohistochemistry ("IHC").

The Ventana Medical Systems' murine antibody clone, 3C6, was used to detect HER1/EGFR expression by immunohistochemistry. The 3C6 clone reacts with the extra-cellular domain of the receptor. Other biomarkers investigated were pHER1, PTEN, pAKT, pMEK, Ki67, and pERK. pTYR was also assessed as a surrogate of activation. All reagents were used as described below and in **TABLE 1,** and according to specified package inserts.

The performance of probes and antibodies to detect protein markers in automated IHC protocols was evaluated in FFPE single slide sections, and in a multi-tissue array (see **TABLE 2).** All IHC analyses were carried out on Ventana's BenchMark®XT and/or Discovery®XT staining platform.

**TABLE 1. Histochemistry Protocols.**

| **IHC Assays** | **Vendor** | **Catalog No** | **Source** |
|---|---|---|---|
| HER1, clone 3C6 | Ventana | 790-2988 | mouse monoclonal |
| EGFR vIII (806) | Ludwig Inst. | Na | mouse monoclonal |
| pHER1 (tyr 1068) | CST | 2236 | mouse monoclonal |
| PTEN | CST | 9559 | rabbit monoclonal |
| pAKT (ser 473) | CST | 3787 | rabbit monoclonal |
| pMEK (ser 217/221) | CST | 9121 | rabbit polyclonal |
| Ki67 | Ventana | 790-2910 | mouse monoclonal |
| pERK (pTpY 185/187) | Ventana | 760-4230 | rabbit polyclonal |
| pTYR clone 4G10 | Upstate | 05-321 | mouse monoclonal |

For the single slide sections, cells were harvested and fixed in 10% neutral buffered formalin ("NBF") and then paraffin-embedded ("FFPE"). FFPE cells were centrifuged for 10 min at 1500 rpm. Supernatant was removed and 3 drops of reagent 1 of the Shandon Cytoblock® Cell Block Preparation System ("Shandon Cytoblock") (Thermo Electron Corporation, Waltham, MA) was added. Cells were centrifuged for 2 min at 3000 rpm. Three (3) drops of Shandon Cytoblock reagent 2 were dripped down the side of tube to allow reagent 2 to flow under the cell pellet suspension. Samples were incubated for 10 min, and then 5 ml of 70% ethanol was added (pellet floated to top of ethanol). Finally, samples were spun for 2 min at 3000 rpm, then transferred to a biopsy cassette and processed for paraffin embedding.

Hematoxylin and Eosin ("H&E") staining was reviewed to verify suitability of the sections for IHC and *in situ* hybridization (ISH) (**FIGURE 3**). H&E staining comprised the following steps: deparaffinizing in xylene, 100% ethanol and 95% ethanol, then immersion in water. Slides were immersed in hematoxylin for 3 min, rinsed in water, immersed in bluing reagent for 1 min, rinsed in water, dipped in eosin and finally a coverslip was added.

Immunoassays involved the following steps: antigen unmasking, and detection subsequent to incubation with the relevant primary and secondary antibodies. As a negative control, either the BenchMark XT® or Discovery XT® Diluent (Ventana) was incubated with the relevant slides. Primary antibodies were detected using the DABMap™, OmniMap™ (Discovery XT®), or iView™ DAB (BenchMark XT®) detection kit according to the manufacturer's instructions. Briefly, iVIEW™ DAB Detection Kit detected specific mouse IgG, IgM and rabbit IgG antibodies bound to an antigen in paraffm-embedded or frozen tissue section. The specific antibody was located by a biotin-conjugated secondary antibody. This step was followed by the addition of a streptavidin-enzyme conjugate that bound the biotin present on the secondary antibody. The complex was then visualized utilizing a precipitating chromogenic enzyme product.
At the end of each incubation step, the automated slide stainer washed the sections to remove unbound material and applied a liquid coverslip that minimized evaporation of aqueous reagents from the slide. Results were interpreted using a light microscope and aided in the differential diagnosis of pathophysiological processes, which may or may not have been associated with a particular antigen.

As a specific example, the detection of pMEK was accomplished in the following manner. H&E's were reviewed by a pathologist to verify tumor presence for tissues and cell viability for cell lines and tissues. Primary antibody pMEK was obtained from Cell Signaling Technology, Inc. ("CST") (Danvers, MA).

For the pMEK IHC assay, cell conditioning was carried out on a Ventana Benchmark® series instrument with CC1 conditioning buffer for 60 minutes at 100°C, where CC1 is a high pH cell conditioning solution: TrisBorate/EDTA buffer, pH8 (Ventana). Slides were incubated with a 1/40 dilution of the stock concentration of the primary pMEK antibody (**TABLE 1**) for 1 hour at room temperature. Stock antibody concentration refers to the concentration at which the antibody is sold commercially; this information is not made available by some manufacturers and appropriate dilutions are determined experimentally. As a negative control, Ventana antibody diluent, used in accordance with manufacture's instructions, was incubated with the relevant slides under the same conditions. pMEK antibody was detected using the Ventana iView DAB detection kit with the exception of the universal secondary antibody, which was replaced by the Vector biotinylated anti-rabbit IgG, according to the manufacturer's instructions (Vector Laboratories, Burlingame, CA) and applied for 32 minutes at 37°C. Enzymatic detection/localization of pMEK was accomplished with a streptavidin horseradish peroxidase conjugate (Ventana), followed by reaction with hydrogen peroxide in the presence of diaminobenzidine ("DAB") and copper sulfate, according to the manufacture's instructions and the kit used (see Table 1). The conjugate and all chromogenic reagents, with the exception of the Vector biotinylated secondary rabbit antibody, are also components of the iView detection kit and were applied at times recommended by the manufacturer.

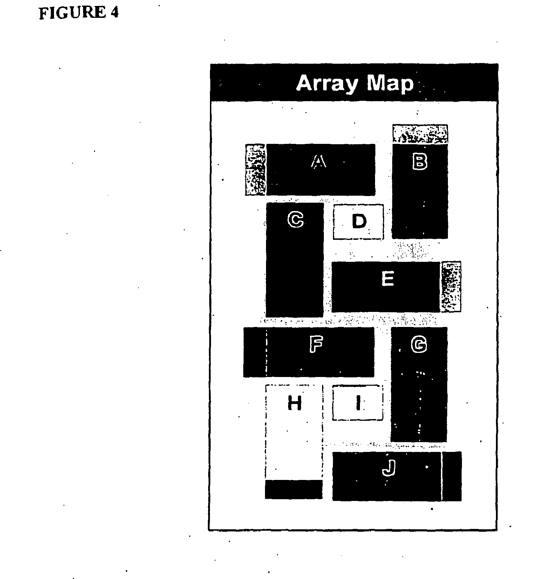

The colorectal progression tissue microarray (TMA; CHTN2003CRCProg) was obtained from the Cooperative Human Tissue Network (CHTN). Details of the array are shown in **FIGURE 4**, and are summarized in **TABLE 2**. Briefly, this colorectal cancer progression array of formalin-fixed and paraffin-embedded ("FFPE") was created by collecting samples from donors. This TMA represents a limited number of cases that may detect strong trends in differential gene expression. Unstained histologic sections were 4 microns thick, and provided on charged glass slides. CHTN2003CRCprog TMA contains up to 20 cases of non-neoplastic colonic mucosa, 14 cases of adenomatous polyps, 14 cases of primary colorectal adenocarcinomas, 7 cases of adenocarcinoma metastatic to regional lymph nodes and 7 cases of adenocarcinoma metastatic to distant sites. Each case is sampled three times with 0.6 mm cores.

**TABLE 2. Composition of the CHTN Colorectal Progression Array.**

| **Code** | **Tissue type** | **# of Cases** |
|---|---|---|
| **C** | adenomas, ≤ 2cm in maximum dimension | 7 |
| **F** | adenomas, > 2 cm in maximum dimension | 7 |
| **E** | primary invasive adenocarcinoma, pathologic stage T1 or T2 | 7 |
| **H** | primary invasive adenocarcinoma, pathologic stage T3 or T4 | 7 |
| **J** | colorectal adenocarcinoma metastatic to lymph nodes, same cases as CL primary cancers | 7 |
| **B** | colorectal adenocarcinoma metastatic to distant sites | 7 |
| **A** | normal non-neoplastic colonic mucosa from non-cancer cases | 7 |
| **G** | normal non-neoplastic colonic mucosa from cancer cases | 7 |
| **D** | inflamed and or regenerative non-neoplastic mucosa (ulcerative colitis) | 3 |
| **I** | inflamed and or regenerative non-neoplastic mucosa (ulcerative colitis) | 3 |

Manual Scoring was conducted by board-certified pathologists. Staining intensities, percentage of reactive cells, and cellular localization were recorded. For pathologist evaluations of IHC, scores for stain intensity ranged from, 0 (negative) to 3+ (most positive).

Optical imaging utilized a digital application with image quantification based on the intensity (expressed as average optical density, or avg. OD) of the stain converted to a numerical score. A high resolution image was captured for each sample and the OD value was based on specific classifiers for color range for positively stained cells. Images for analysis were captured using a 40x objective. In some cases a "combined score" or multiplicative index was derived that incorporates both the percentage of positive cells and the staining intensity according to the following formula: *combined score = (% positive) X (optical density score).*

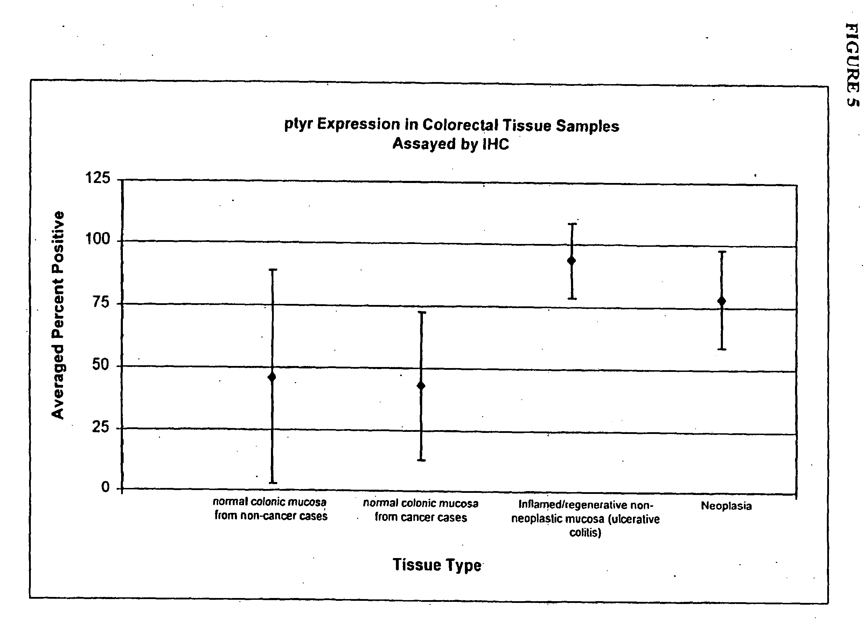

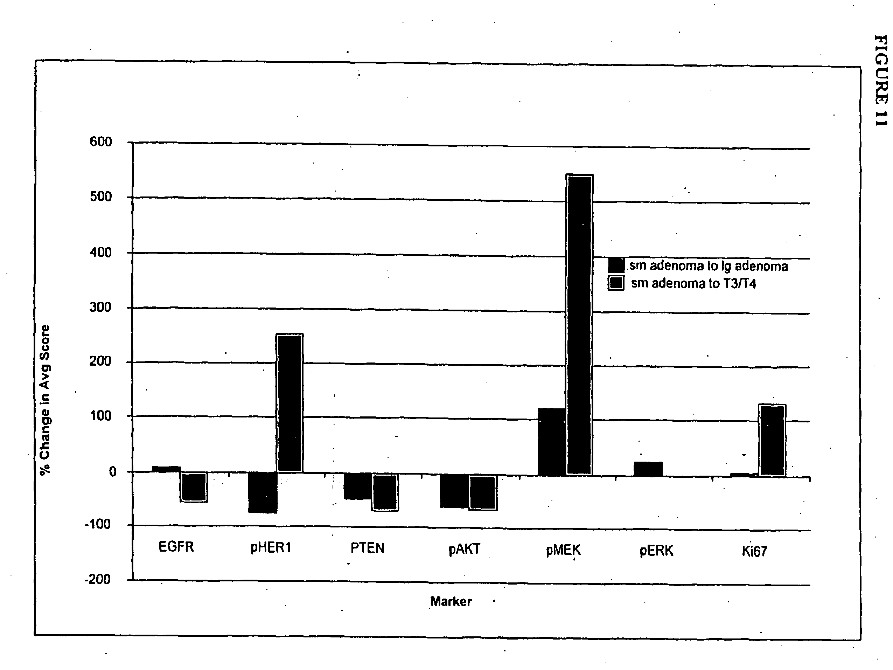

The results of the histochemistry tissue assessment using ptyr activity as a surrogate measure of activation showed an increase in expression in neoplasia and inflamed non-neoplasic tissue as compared to normal colonic mucosa from cancer and non-cancer cases **(****FIGURE 5****).** The results of the histochemistry assessment of expression levels from EGFR pathway molecules in the TMA are shown in **FIGURES 6-9****.** The results of the histochemistry assessment of expression levels from EGFR pathway molecules in the individual cases are shown in **FIGURE 10****.** The results from both experiments show that as colorectal cancer progresses through the sequential staging categories pMEK, Ki67, and pHER1 protein levels increase and EGFR, PTEN, and pAKT protein levels decrease **(****FIGURE 11****).** These findings identify biomarkers profiles that could be useful in diagnosis and tracking of colorectal tumor progression, including high levels of EGFR (protein), PTEN and low levels of pMEK in early small adenomas and low levels of PTEN and high levels of pMEK in advanced diseases states including malignant phenotypes.

### Example 2

### Correlation Between EGFR Gene Copy Number and Colorectal Cancer Tumor Staging using in situ Hybridization

Fluorescent *in situ* hybridization (FISH) for EGFR was preformed on single slide sections from individual cases and a multi-tissue array in order to assess gene status in colorectal cancer progression.

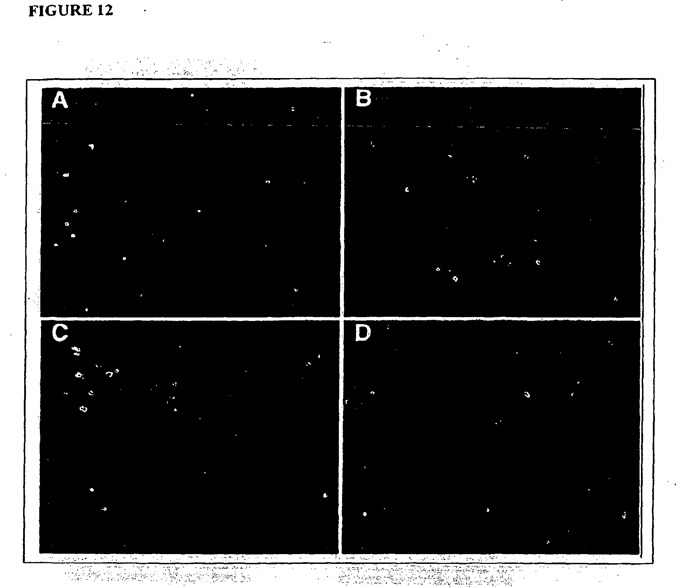

Using dual color FISH, the number of EGFR gene copies per cell was evaluated in formalin-fixed, paraffin-embedded (FFPE) single-slide sections and in a multi-tissue array (**FIGURE 12**). The details of the single slide sections and multi-tissue array are outlined in Example 1.

*In-situ* hybridization detection of the EGFR gene was conducted with either probes from, Ventana (Spectrum Orange labeled), Invitrogen (SPOTLight™ - EGFR, DIG labeled), or Vysis (Spectrum Orange EGFR and Spectrum Green CEP 7 labeled probes). The EGFR probes from Ventana and Zymed were detected with fully automated protocols on the Ventana Discovery®XT. Detection of the Vysis probes was semi-automated, with probe hybridization conducted offline as described by the manufacturer (Vysis, Downers Grove, IL). The FISH protocol was performed according to the manufactures package insert (Vysis, Cat. No. 32-191053). FISH evaluation was conducted as described in Hirsch et .al. JCO, 21: 3798-3807). Figure 12 shows representative photomicrographs from cells that display balanced disomy, balanced trisomy, balanced polysomy, and gene amplification.

Average numbers of gene copies per cell were determined for EGFR and CEP7 by pathology review. The results of the EGFR FISH assays in individual cases and in the TMA demonstrate that EGFR gene status evolves from balanced disomy (normal) to balanced trisomy/polysomy (abnormal) from small adenoma to adenocarcinoma **(****FIGURES 13****)** as shown in **TABLE 3.** Samples with elevated levels of EGFR gene copies (trisomy+) all had sub-populations with high levels of protein as assayed by IHC (3+, > 50%) (Example 1). The findings of Example 1 and 2 also suggest discordant levels of gene amplification (normal) and protein expression (high) in early cancer stages.

**TABLE 3. Summary Distribution of EGFR Gene Expression in Colorectal Cancer Progression.**

| **FISH Pattern** | | | | | | |
|---|---|---|---|---|---|---|
| Diagnosis | No.of Cases | Balanced disomy | Balanced trisomy | Balanced polysomy | GA-low level | GA-high level |
| Adenomas less than 2cm | 7 | 100% | - | - | - | - |
| Adenomas greater than 2cm | 7 | 86% | 14% | - | - | - |
| Primary invas, adenoca stage T1 or T2 | 6 | 83% | 17% | - | - | - |
| Primary invas, adenoca stage T3 or T4 | 7 | 86% | - | 14% | - | - |
| Adenoca metastatic to LN | 6 | 67% | - | 33% | - | - |
| Adenoca metastatic to distant sites | 7 | 86% | - | - | 14% | |

### Example 3 Heterogeneity in Expression Levels of EGFR Pathway Molecules within a Tumor

The expression levels of EGFR pathway molecules were assessed in colorectal cancer case study to evaluate possible heterogeneity in the expression levels of these molecules in a single tumor.

A 41 year old man presenting with rectal bleeding and abdominal cramping was diagnosied with a 3 cm rectal mass. Distal colon resection after radiotherapy revelaed a well differentiated rectal adeno-carcinoma of stage T3. Single slide sections from various regions of the tumor were prepared as detailed in Example 1. IHC was performed to determine expression levels for EGFR, HER-2, pAKT, Ki67, Survivin and VEGF as described in Example 1. Survivan was detected with an antibody from Novus (NB500-201) by incubating for 2 hrs at room temperature. VEGF was detected using an antibody from Santa Cruz (SC-7269) by including for 1 hr at room temperature.

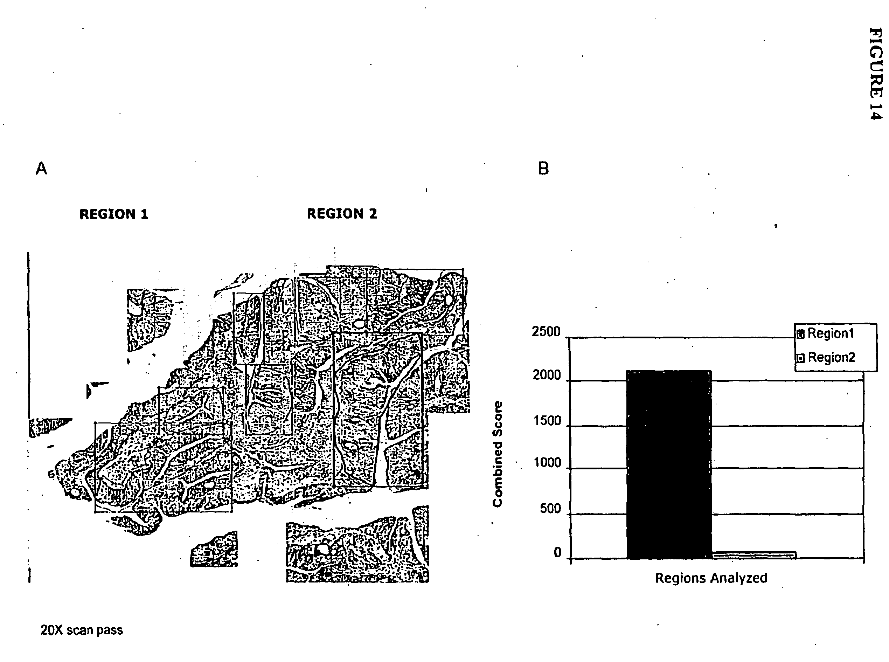

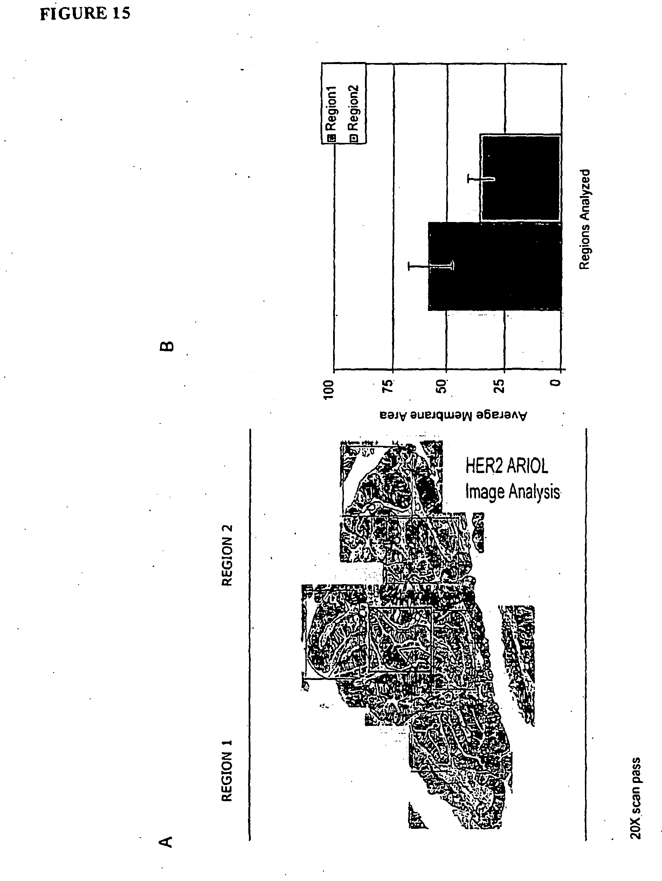

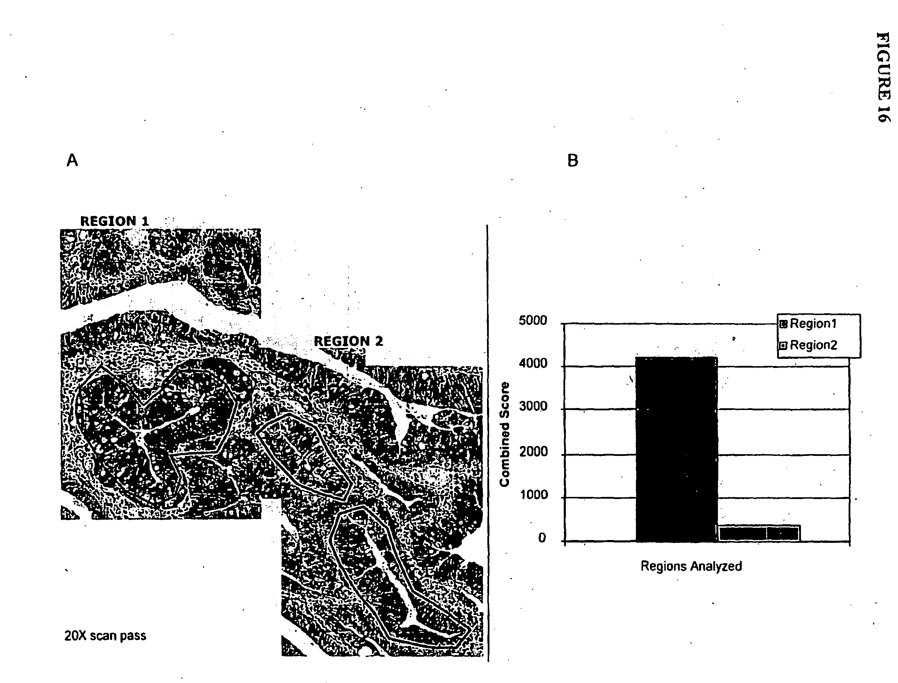

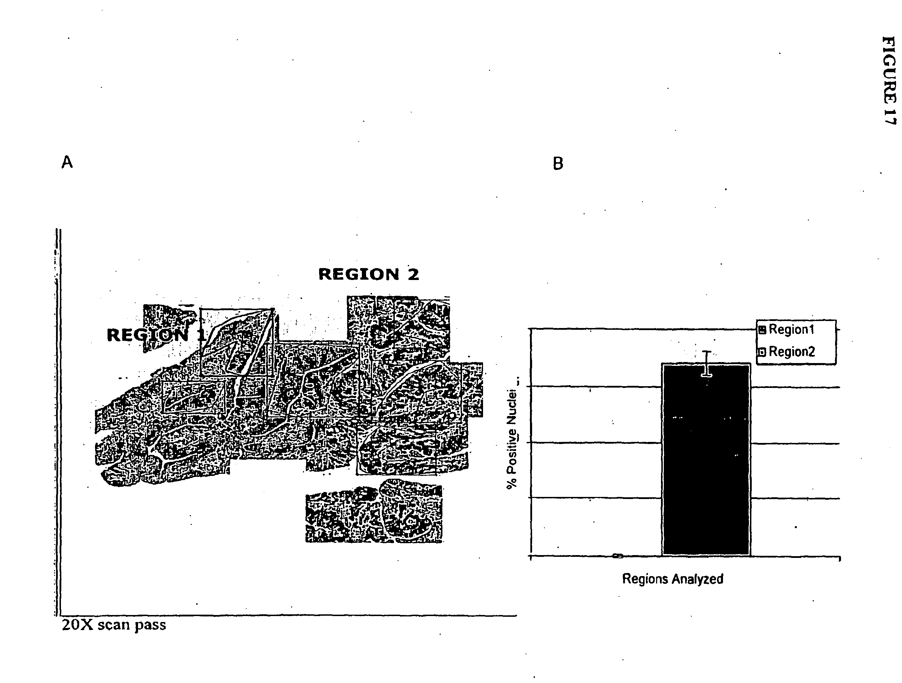

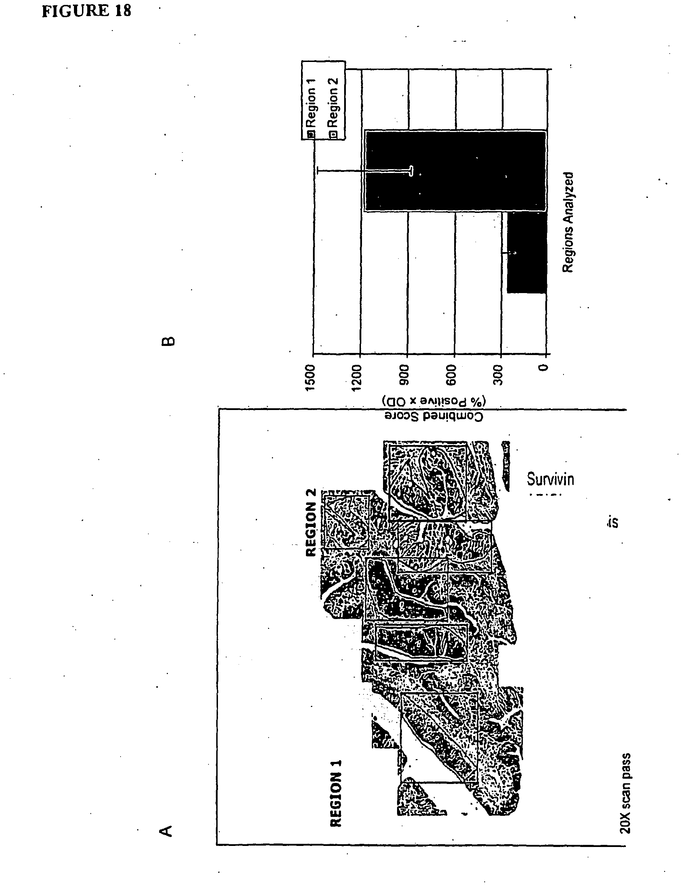

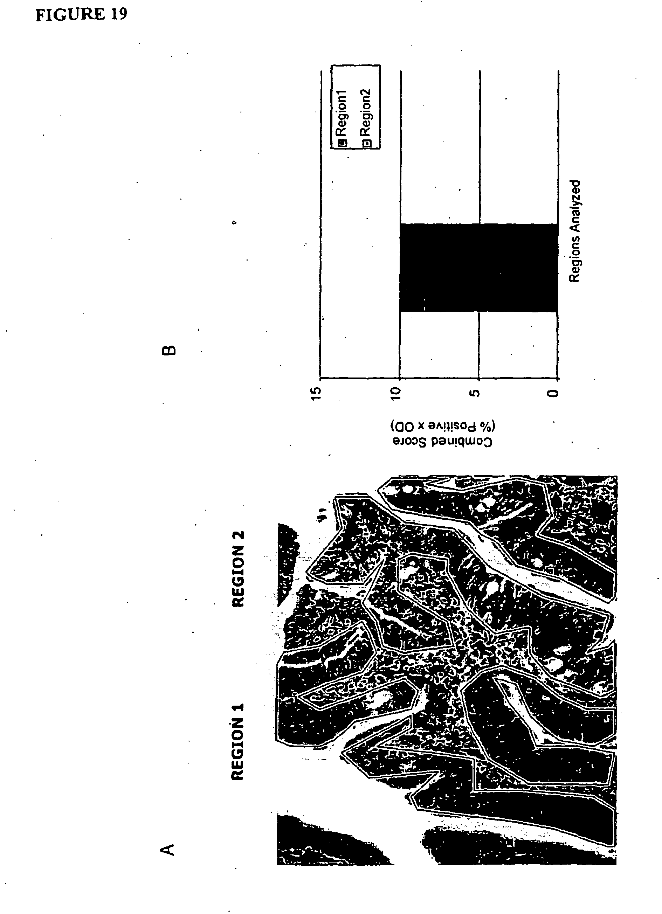

**FIGURES 14-19** show that expression levels of tumor bio-markers can vary significantly within the same tumor. These results demonstrate that patients may benefit from individualized combination therapies tailored toward the individual expression signature of the particular tumor.

It should be understood that the foregoing disclosure emphasizes certain specific embodiments of the invention and that all modifications or alternatives equivalent thereto are within the spirit and scope of the invention as set forth in the appended claims.

## Claims

1. A method for identifying a colorectal cancer tumor responsive to a chemotherapeutic agent, comprising assaying a tissue or cell sample obtained from the colorectal cancer tumor to detect a pattern of expression, phosphorylation, or both expression and phosphorylation of one or more biological markers, wherein the biological markers are EGFR, PTEN, pAKT, pMEK, pHER1, pERK, or Ki67, wherein the expression, phosphorylation or both expression and phosphorylation of the biological markers identifies the mammalian tumor as being treatable with a chemotherapeutic agent.

2. The method of claim 1, wherein the chemotherapeutic agent is an EGFR antibody.

3. The method of claim 1, wherein the chemotherapeutic agent is a kinase inhibitor.

4. The method of claim 1, wherein the chemotherapeutic agent is both an EGFR antibody and a kinase inhibitor.

5. The method of claim 1, wherein the method consists of assaying a tissue or cell sample obtained from the individual to detect a pattern of expression, phosphorylation, or both expression and phosphorylation of two or more biological markers.
